# EUROPEAN PATENT APPLICATION

(11) **EP 4 843 627 A2**
(43) Date of publication of application: **25.11.2026**
(21) Application number: 26211155.2
(22) Date of filing: 12.09.2019
(51) Int. Cl.: A61P 21/00

(54) **COSMETIC METHOD TO REDUCE PROMINENCE OF THE MASSETER MUSCLE**

(30) Priority: 13.09.2018 US 201862731064 P
(62) Document number(s) of the earlier application(s) in accordance with Art. 76 EPC: 19778736.9 / 3 849 585
(71) Applicant: Allergan, Inc., North Chicago, IL 60064 (US)
(72) Inventor: LEE, Elisabeth, Laguna Beach, California, 92651 (US); BOWEN, Beta, Santa Ana, California, 92705 (US); SOMOGYI, Christine, Santa Ana, California, 92705 (US); ROGERS, John D., 75004 Paris (FR)
(74) Representative: Hoffmann Eitle

(57) **Abstract**

Methods and kits for treating or alleviating masseter muscle hypertrophy by local administration of a Clostridial derivative, such as a botulinum toxin, to the masseter muscle are described. Methods and kits for reducing lower face width and for reducing prominence of the masseter muscle of a human are also described.

## Description

### CROSS REFERENCE TO RELATED APPLICATIONS

This application claims the benefit of U.S. Provisional Application No. 62/731,064, filed September 13, 2018, incorporated by reference herein in its entirety.

### TECHNICAL FIELD

The present disclosure relates to methods for treatment of masseter muscle hypertrophy. In particular, the present disclosure relates to the treatment of masseter muscle hypertrophy (hereinafter MMH) using neurotoxins.

### BACKGROUND

Masseter muscle hypertrophy (MMH) may be unilateral or bilateral, and it may be idiopathic or occur in association with several conditions such as bruxism, occlusal and muscular imbalances, temporomandibular joint disorder (TMJD), or excessive chewing habits. Aesthetically, MMH may appear as a wide, square, or trapezoidal lower face shape that is deemed undesirable. Individuals may seek surgical or nonsurgical alteration of prominent masseter muscles and/or mandibles to decrease a bulky or square-appearing lower face.

Success of a treatment depends on several factors, including for example effective methods to assess the effects of the treatment so that further treatment may be tailored accordingly, an effective administration paradigm which produces a desired outcome while minimizing adverse events that may be associated with the treatment.

Thus, there is a need for effective treatment methods for MMH as well as effective methods for determining efficacy and safety of such treatment.

### BRIEF SUMMARY

In a first aspect, a method for treating MMH is provided. The method comprises locally administering a clostridial derivative to a subject having MMH. In some embodiments, the clostridial derivative is a native or recombinant neurotoxin, a recombinant modified toxin, fragments thereof, a Targeted vesicular Exocytosis Modulator (TEM), or combinations thereof. In one embodiment, the clostridial derivative is a botulinum toxin. In one embodiment of the method, the clostridial derivative is locally administered into the masseter muscle of the patient.

In yet another aspect, a kit of parts is provided. The kit of parts comprises: a) botulinum toxin in an amount of about 5-100 Units, and b) instructions for administration of botulinum toxin into a muscle for the treatment of masseter muscle hypertrophy, in which administering botulinum toxin into the masseter muscle of a subject having masseter muscle hypertrophy. In one embodiment, the amount of botulinum toxin is between about 10-50 Units or between 20-100 Units, preferably between about 12-48 Units or 24-96 Units, more preferably between about 24-36 Units or 48-72 Units. In a specific embodiment, the amount of botulinum toxin is 12 Units, 24 Units, 36 Units, 48 Units, 72 Units or 96 Units. In one embodiment, the botulinum toxin is packaged in multiple portions.

In another aspect, a method to reduce lower face width in the masseter muscle region is provided. The method comprises identifying a region of maximal bulge of the masseter muscle and injecting botulinum toxin at a plurality of injection sites in the region to administer a dose of botulinum toxin to reduce lower face width in the masseter muscle region.

In one embodiment, the steps of identifying and injecting are performed bilaterally. In another embodiment, the method further comprises repeating the identifying and the injecting on an opposing masseter muscle.

In one embodiment, a dose of botulinum toxin of between about 10-50 Units or between 20-100 Units is injected in the plurality of injection sites. In another embodiment, a dose of botulinum toxin of between about 24-36 Units or 48-72 Units is injected in the plurality of injection sites. In other embodiments, the dose is between about 12-48 Units or 24-96 Units. In other embodiments, the dose is 12 Units, 24 Units, 36 Units, 48 Units, 72 Units or 96 Units. In still other embodiments, the dose of botulinum toxin of between about is in a range selected from the group of ranges of between about 5-100 Units, between about 15 - 60 Units, between about 25-47 Units, and between about 30-40 Units, to each of the muscles treated. In another embodiment, the dose of botulinum toxin to each of the muscles treated is about 36 Units.

In another embodiment, the plurality of injection sites is between 3-5 injection sites. In some embodiments, a portion of the dose is administered at each injection site in the plurality of injection sites, where the portion is equal to the dose divided by the number of sites in the plurality of injection sites. In other embodiments, the portion of the dose administered at one or more of the injection sites is not the same as the portion of the dose administered at another injection site.

In another embodiment, the method comprises, prior to identifying the region of maximal bulge of the masseter muscle, assessing or reviewing an assessment of a subject using a Masseter Muscle Prominence Scale (MMPS). In one embodiment, the assessing or reviewing assigns a rating to the subject of marked or very marked on the MMPS.

In one embodiment, the assessing or reviewing is carried out to evaluate the efficacy of a previous treatment.

In another embodiment, the method is a cosmetic method to reduce lower face width in a masseter muscle region.

In yet another aspect, a kit of parts is provided. The kit of parts comprises: a) botulinum toxin in an amount of about 5-100 Units, and b) instructions for administration of botulinum toxin into a muscle for reducing lower face width in a masseter muscle region, in which i) identifying a region of maximal bulge of the masseter muscle, and ii) injecting botulinum toxin at a plurality of injection sites in the region to administer a dose of botulinum toxin to reduce lower face width in the masseter muscle region. In one embodiment, the amount of botulinum toxin is between about 10-50 Units or between 20-100 Units, preferably between about 12-48 Units or 24-96 Units, more preferably between about 24-36 Units or 48-72 Units. In a specific embodiment, the amount of botulinum toxin is 12 Units, 24 Units, 36 Units, 48 Units, 72 Units or 96 Units. In one embodiment, the botulinum toxin is packaged in multiple portions, for example, 3-5 portions. In one embodiment, the botulinum toxin is botulinum toxin type A. In one embodiment, the kit of parts further comprises instructions for assessing or reviewing an assessment of a subject using a Masseter Muscle Prominence Scale (MMPS) prior to identifying the region of maximal bulge of the masseter muscle, instructions for identifying a region of maximal bulge of the masseter muscle, and/or instructions for repeating the identifying and the injecting on an opposing masseter muscle.

In another aspect, a method to reduce prominence of the masseter muscle of a human is provided. The method comprises administering to a region of the masseter muscle a dose of botulinum toxin that reduces prominence of the masseter muscle, where the dose is administered to a plurality of sites in the region of the masseter muscle. The region is identified as a region of maximal bulge in the masseter muscle when the jaw is in a clenched position and the dose is administered with the jaw in a relaxed position.

In one embodiment, the step of administering is performed bilaterally. In another embodiment, the method further comprises repeating the administering on an opposing masseter muscle.

In one embodiment, the administering step is by injection with a needle positioned during the injection to be perpendicular to the masseter muscle.

In another embodiment, the dose is administered in a manner for distribution to deep muscle and to superficial muscle.

In still another embodiment, the dose is administered in a volume of between about 0.3-3.6 mL. In another embodiment, the dose is administered in a volume of between about 0.6-2.4 mL.

In yet another embodiment, a portion of the dose is administered at each injection site in the plurality of injection sites, where the portion administered at each injection site is unequal. In one embodiment, the portion, whether equal at each injection site or unequal at each injection site, is administered in a volume of between about 0.1-0.4 mL.

In one embodiment, the plurality of injection sites is 3 injection sites. In other embodiments, the plurality of injection sites is between 2-5 or 3-5.

In one embodiment, at least one site in the plurality of injection sites is spaced from an adjacent site in the plurality of injection sites by about 1 cm. In another embodiment, each injection site in the plurality of injection sites is spaced from an adjacent site by about 0.5 cm, 0.75 cm, or 1 cm. In one embodiment, each injection site is spaced from an adjacent site by about 1 cm. In the studies described in Examples 1 and 2, it was observed that 1cm spacing between the injection sites provided a uniform and contiguous toxin distribution across the treatment area.

In another aspect, a kit of parts is provided. The kit of parts comprises: a) botulinum toxin in an amount of about 5-100 Units, and b) instructions for administration of botulinum toxin into a muscle for reducing prominence of the masseter muscle of a human, in which i) identifying a region of maximal bulge in the masseter muscle when the jaw is in a clenched position, and ii) when the jaw is in a relaxed position, administering to a plurality of sites in the region of the masseter muscle a dose of botulinum toxin that reduces prominence of the masseter muscle. In one embodiment, the amount of botulinum toxin is between about 10-50 Units or between 20-100 Units, preferably between about 12-48 Units or 24-96 Units, more preferably between about 24-36 Units or 48-72 Units. In a specific embodiment, the amount of botulinum toxin is 12 Units, 24 Units, 36 Units, 48 Units, 72 Units or 96 Units. In one embodiment, the botulinum toxin is botulinum toxin type A. In one embodiment, the botulinum toxin is packaged in multiple portions, preferably 2-5 portions, more preferably 3 portions.

In yet another aspect, a method to temporarily reduce lower face convexity or width associated with masseter muscle prominence is provided. The method comprises identifying a line that extends from the lateral commissure of the mouth to the point where the ear lobe attaches to the face; identifying a region of maximal bulge of the masseter muscle with the jaw in a clenched position; determining a treatment area that includes the region of maximal bulge, is positioned at or below the line, is posterior to the risorius muscle and is anterior to the parotid gland; and injecting botulinum toxin at a plurality of injection sites in the treatment area to administer a dose of botulinum toxin that reduces lower face convexity or width.

In one embodiment, the steps of the method are performed bilaterally. In another embodiment, the method further comprises repeating the steps on an opposing masseter muscle.

In one embodiment, the line that extends from the lateral commissure of the mouth to the point where the ear lobe attaches to the face is identified visually as an imaginary line. In other embodiments, the line is visually identified and physically marked on the skin of the patient.

In one embodiment, the treatment area that is determined visually without marking skin. In other embodiments, the treatment area that is determined is denoted with a marking on the skin.

In other embodiments, an assessment of the subject using a Masseter Muscle Prominence Scale (MMPS) is conducted, or a prior assessment of the subject is reviewed. In one embodiment, the subject to be treated has a rating according to the MMPS of marked or very marked.

In one embodiment, the assessing or reviewing is carried out to evaluate the efficacy of a previous treatment.

In another embodiment, the method is a cosmetic method to temporarily reduce lower face convexity or width associated with masseter muscle prominence.

In another embodiment, the botulinum toxin is botulinum toxin type A.

In another aspect, a kit of parts is provided. The kit of parts comprises: a) botulinum toxin in an amount of about 5-100 Units, and b) instructions for administration of botulinum toxin into a muscle for temporarily reducing lower face convexity or width associated with masseter muscle prominence, in which i) identifying a line that extends from the lateral commissure of the mouth to the point where the ear lobe attaches to the face; ii) identifying a region of maximal bulge of the masseter muscle with the jaw in a clenched position; iii) determining a treatment area that includes the region of maximal bulge, is positioned at or below the line, is posterior to the risorius muscle and is anterior to the parotid gland; and iv) injecting botulinum toxin at a plurality of injection sites in the treatment area to administer a dose of botulinum toxin that reduces lower face convexity or width. In one embodiment, the amount of botulinum toxin is between about 10-50 Units or between 20-100 Units, preferably between about 12-48 Units or 24-96 Units, more preferably between about 24-36 Units or 48-72 Units. In a specific embodiment, the amount of botulinum toxin is 12 Units, 24 Units, 36 Units, 48 Units, 72 Units or 96 Units. In one embodiment, the botulinum toxin is packaged in multiple portions, preferably 2-5 portions, more preferably 3 portions. In one embodiment, the botulinum toxin is botulinum toxin type A. In one embodiment, the kit of parts further comprises instructions for assessing or reviewing an assessment of a subject using a Masseter Muscle Prominence Scale (MMPS), which assigns a rating to the subject of marked or very marked on the MMPS.

In another aspect, a method to alter the contour of a lower face of a human is provided. The method comprises locally administering a clostridial derivative. In other embodiments, the method comprises identifying a region of maximal bulge of the masseter muscle and injecting a dose of clostridial derivative at a plurality of injection sites in the region to administer to reduce lower face width in the masseter muscle region. In one embodiment, the clostridial derivative is a botulinum toxin. In still other embodiments, the method comprises identifying a line that extends from the lateral commissure of the mouth to the point where the ear lobe attaches to the face; identifying a region of maximal bulge of the masseter muscle with the jaw in a clenched position; determining a treatment area that includes the region of maximal bulge, is positioned at or below the line, is posterior to the risorius muscle and is anterior to the parotid gland; and injecting botulinum toxin at a plurality of injection sites in the treatment area to administer a dose of botulinum toxin that reduces lower face convexity or width.

In another aspect, a kit of parts is provided. The kit of parts comprises: a) botulinum toxin in an amount of about 5-100 Units, and b) instructions for administration of botulinum toxin into a muscle for altering the contour of a lower face of a human, in which i) identifying a line that extends from the lateral commissure of the mouth to the point where the ear lobe attaches to the face; ii) identifying a region of maximal bulge of the masseter muscle with the jaw in a clenched position; iii) determining a treatment area that includes the region of maximal bulge, is positioned at or below the line, is posterior to the risorius muscle and is anterior to the parotid gland; and iv) injecting botulinum toxin at a plurality of injection sites in the treatment area to administer a dose of botulinum toxin that reduces lower face convexity or width. In one embodiment, the amount of botulinum toxin is between about 10-50 Units or between 20-100 Units, preferably between about 12-48 Units or 24-96 Units, more preferably between about 24-36 Units or 48-72 Units. In a specific embodiment, the amount of botulinum toxin is 12 Units, 24 Units, 36 Units, 48 Units, 72 Units or 96 Units. In one embodiment, the botulinum toxin is packaged in multiple portions, preferably 2-5 portions, more preferably 3 portions. In one embodiment, the botulinum toxin is botulinum toxin type A.

In still another aspect, a method to sculpt or reshape a lower face of a human in the masseter muscle region is provided. Another aspect contemplates a method to reduce lower face convexity associated with the masseter muscle. In embodiments of these methods, a clostridial derivative is locally administered according to any of the methods and embodiment described herein.

In one embodiment of any of the methods or kit of parts, the method or composition is intended for a cosmetic method of treatment, e.g., a cosmetic method to reduce prominence of the masseter muscle of a human, a cosmetic method to temporarily reduce lower face convexity or width associated with masseter muscle prominence, or a cosmetic method to reduce lower face width in a masseter muscle region.

In another aspect, a kit of parts is provided. The kit of parts comprises: a) botulinum toxin in an amount of about 5-100 Units, and b) instructions for administration of botulinum toxin into a muscle for sculpting or reshaping a lower face of a human in the masseter muscle region. In one embodiment, the amount of botulinum toxin is between about 10-50 Units or between 20-100 Units, preferably between about 12-48 Units or 24-96 Units, more preferably between about 24-36 Units or 48-72 Units. In a specific embodiment, the amount of botulinum toxin is 12 Units, 24 Units, 36 Units, 48 Units, 72 Units or 96 Units. In one embodiment, the botulinum toxin is packaged in multiple portions, preferably 2-5 portions, more preferably 3 portions. In one embodiment, the botulinum toxin is botulinum toxin type A.

In any of the foregoing methods or kit of parts, the botulinum toxin is animal protein free in some embodiments. In other embodiments, the lower face width in a masseter muscle region, the prominence of the masseter muscle of a human, or the lower face convexity or width associated with masseter muscle prominence is reduced for a period of time longer than obtained with an animal-protein containing composition.

In still another other aspect, a composition comprising botulinum toxin and a pharmaceutically acceptable carrier for injection into a region of maximal bulge of the masseter muscle to reduce lower face width in the masseter muscle region of a subject is provided.

In yet another aspect, use of botulinum toxin for injection into a region of maximal bulge of the masseter muscle to reduce lower face width in the masseter muscle region of a subject, to reduce prominence of the masseter muscle of a human, and/or to temporarily reduce lower face convexity or width associated with the masseter muscle prominence a subject, is provided.

In one embodiment of the composition or its use, the subject has marked or very marked masseter muscle prominence. In one embodiment, the masseter muscle prominence is determined using the MMPS.

In another embodiment, the composition or its use provides a reduction in lower face width in the masseter muscle region for 90 days after injection.

In other embodiments, the composition or its use of the composition is injected bilaterally to the masseter muscles at a plurality of injection sites at each masseter muscle. In one embodiment, the plurality of injection sites is between 3-5 per masseter muscle.

In other embodiments, the composition or its use of the composition is injected with a needle positioned during injecting to be perpendicular to the masseter muscle. In other embodiments, the composition is injected in a manner for distribution to deep muscle and to superficial muscle.

In yet other embodiments, composition or its use is intended for a cosmetic treatment.

The composition or it use, in one embodiment, is where the botulinum toxin is botulinum toxin type A. In one embodiment, the botulinum toxin is animal protein free.

In one embodiment, the lower face width in a masseter muscle region, the prominence of the masseter muscle of a human, or the lower face convexity or width associated with masseter muscle prominence is reduced for a period of time longer than obtained with an animal-protein containing composition.

### BRIEF DESCRIPTION OF THE FIGURES

The following drawings are presented to illustrate aspects and features of embodiments of the present methods.

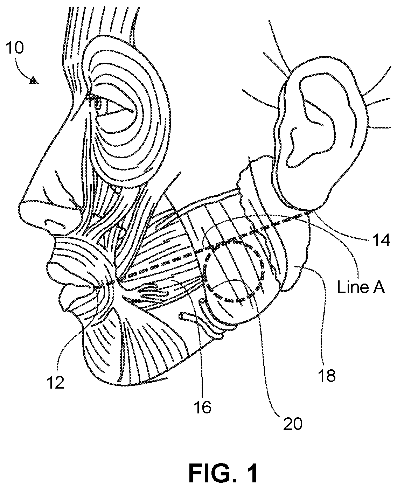

FIG. 1 is a schematic of side view of a human face indicating an approach to identify a treatment area;

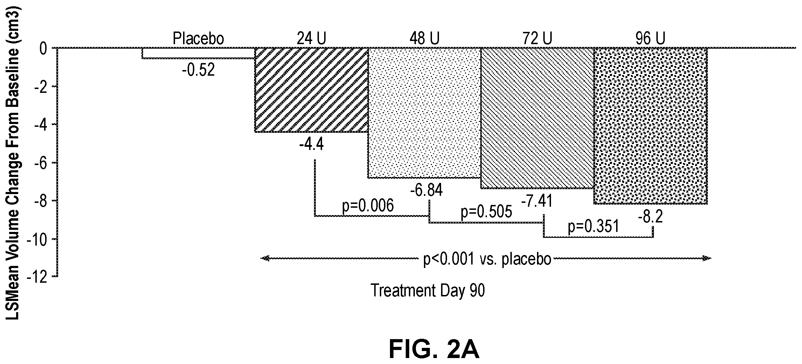

FIG. 2A is a bar graph showing the least squares mean (LSMean) change in lower face volume (in cm³) 90 days after treatment with the indicated dose of botulinum toxin in the masseter muscle, the volume change at day 90 day relative to lower face volume prior to treatment (baseline), where lower face volume is calculated using a three-dimensional imaging system;

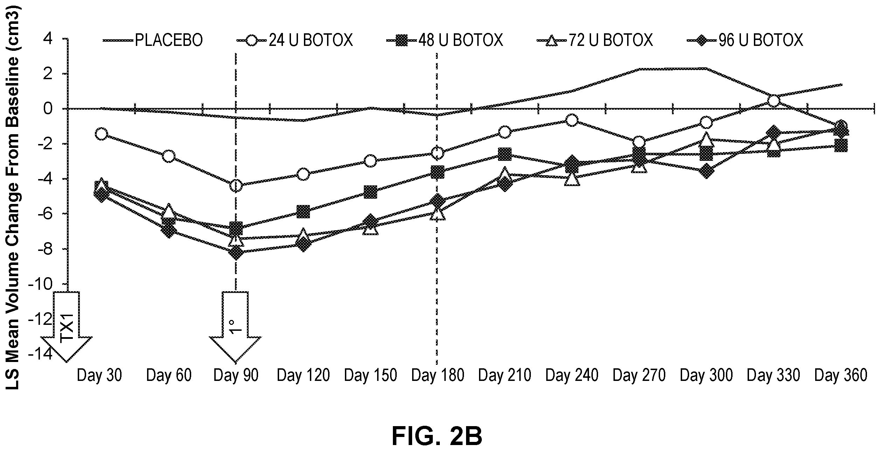

FIG. 2B is a graph showing the least squares mean (LSMean) change in lower face volume (in cm³) as a function of time after treatment with botulinum toxin in the masseter muscle, the volume change at each time point relative to lower face volume prior to treatment (baseline), where lower face volume is calculated using a three-dimensional imaging system, and where a bilateral botulinum toxin dose of 24 units (circles), 48 units (squares), 72 units (triangles), and 96 units (diamonds) was given;

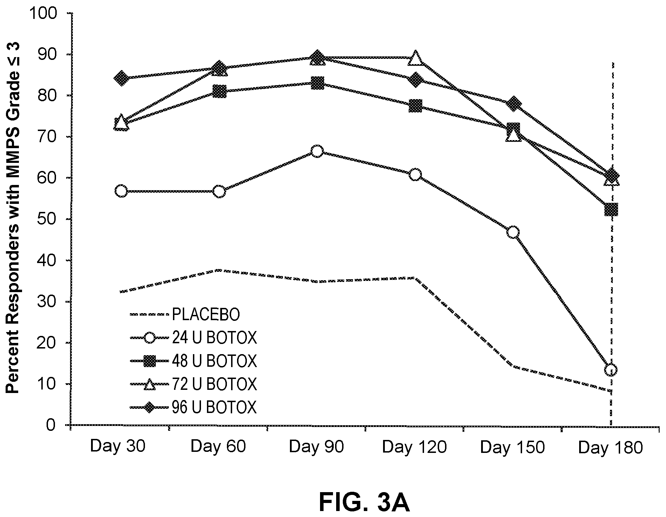

FIG. 3A is a graph showing the percent of subjects in each treatment dose cohort that achieved a rating of Grade 3 or less according to the Masseter Muscle prominence scale (MMPS) as a function of time in the untreated placebo subjects (dashed line) and after treatment with a total bilateral botulinum toxin dose of 24 units (circles), 48 units (squares), 72 units (triangles), and 96 units (diamonds);

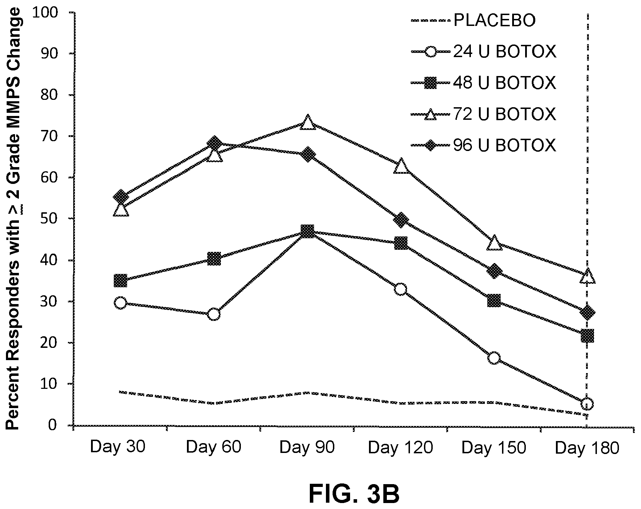

FIG. 3B is a graph showing the percent of subjects in each treatment dose cohort that achieved a change in rating of 2 or more according to the MMPS as a function of time in the untreated placebo subjects (dashed line) and after treatment with a total bilateral botulinum toxin dose of 24 units (circles), 48 units (squares), 72 units (triangles), and 96 units (diamonds);

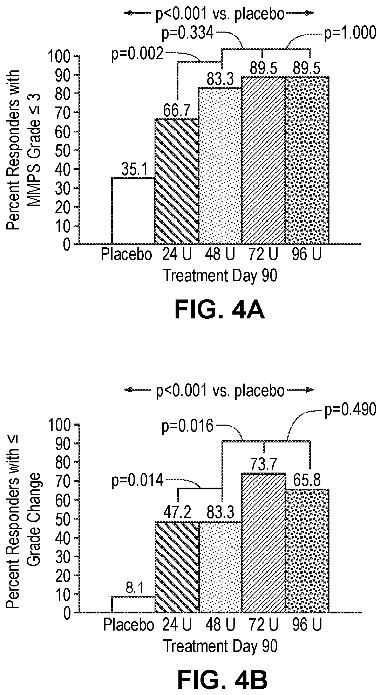

FIG. 4A is a bar graph showing the percent of subjects in each treatment dose cohort that achieved a rating of Grade 3 or less according to the MMPS 90 days after treatment with a bilateral botulinum toxin dose of 24 units, 48 units, 72 units, and 96 units and in untreated placebo subjects;
FIG. 4B is a bar graph showing the percent of subjects in each treatment dose cohort that achieved a change in rating of 2 or more according to the MMPS 90 days after treatment with a bilateral botulinum toxin dose of 24 units, 48 units, 72 units, and 96 units and in untreated placebo subjects;

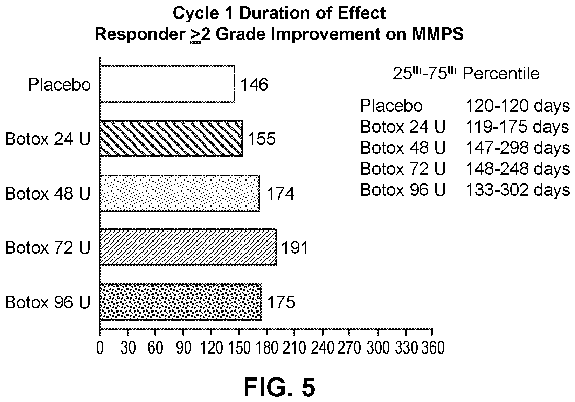

FIG. 5 is a bar graph showing the duration of effect in the subjects of each treatment dose cohort that achieved a change in ranking of 2 or more according to the MMPS after treatment with a bilateral botulinum toxin dose of 24 units, 48 units, 72 units, and 96 units and in untreated placebo subjects;

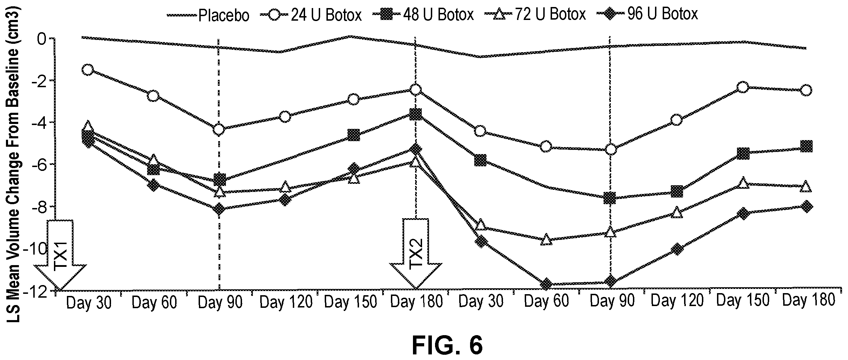

FIG. 6 is a graph showing the least squares mean (LSMean) change in lower face volume (in cm³) as a function of time after treatment with botulinum toxin in the masseter muscle, the volume change at each time point relative to lower face volume prior to treatment (baseline), where lower face volume is calculated using a three-dimensional imaging system, and where a bilateral botulinum toxin dose was given at Day 1 and at Day 180 at doses of 24 units (circles), 48 units (squares), 72 units (triangles), and 96 units (diamonds) was given; and

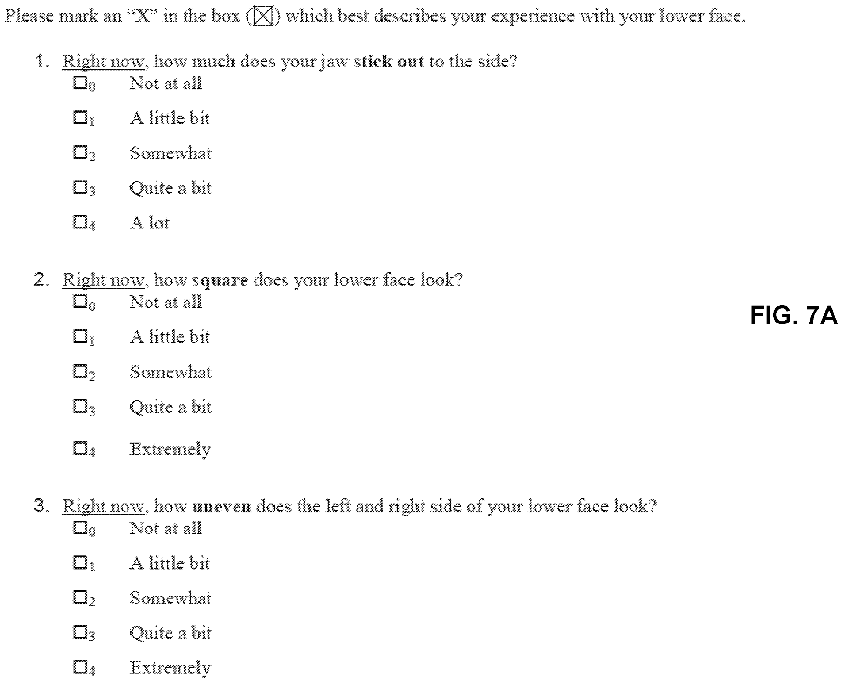

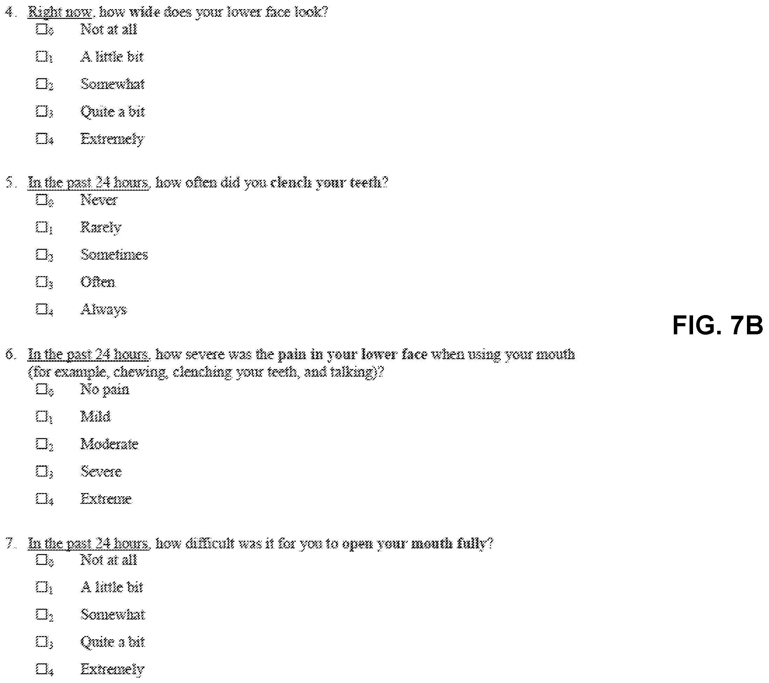

FIGS. 7A-7B present questions on a questionnaire for evaluation of lower facial shape, the Lower Facial Shape Questionnaire.

### DETAILED DESCRIPTION

### DEFINITIONS

"About" or "approximately" as used herein means within an acceptable error range for the particular value as determined by one of ordinary skill in the art, which will depend in part on how the value is measured or determined, (*i.e.*, the limitations of the measurement system). For example, "about" can mean within 1 or more than 1 standard deviations, per practice in the art. Where particular values are described in the application and claims, unless otherwise stated, the term "about" means within an acceptable error range for the particular value.

"Administration", or "to administer" means the step of giving (*i.e*. administering) a botulinum toxin to a subject, or alternatively a subject receiving a pharmaceutical composition. The present method can be performed via administration routes including intramuscular, non-intramuscular, intradermal, subcutaneous administration, transdermal, implantation (for example, of a slow-release device such as polymeric implant or miniosmotic pump), or combinations thereof.

"Alleviating" means a reduction in the occurrence of a symptom. Thus, alleviating includes some reduction, significant reduction, near total reduction, and total reduction. An alleviating effect may not appear clinically for between 1 to 7 days after administration of a clostridial derivative to a patient or sometime thereinafter.

"Animal protein free" means the absence of blood derived, blood pooled and other animal derived products or compounds. "Animal" means a mammal (such as a human), bird, reptile, fish, insect, spider or other animal species. "Animal" excludes microorganisms, such as bacteria. Thus, an animal protein free pharmaceutical composition can include a botulinum neurotoxin. For example, an "animal protein free" pharmaceutical composition means a pharmaceutical composition which is either substantially free or essentially free or entirely free of a serum derived albumin, gelatin and other animal derived proteins, such as immunoglobulins. An example of an animal protein free pharmaceutical composition is a pharmaceutical composition which comprises or which consists of a botulinum toxin (as the active ingredient) and a suitable polysaccharide as a stabilizer or excipient.

"Botulinum toxin" means a neurotoxin produced by Clostridium botulinum, as well as a botulinum toxin (or the light chain or the heavy chain thereof) made recombinantly by a non-Clostridial species. The term "botulinum toxin", as used herein, encompasses Botulinum toxin serotype A (BoNT/A), Botulinum toxin serotype B (BoNT/B), Botulinum toxin serotype C (BoNT/C), Botulinum toxin serotype D (BoNT/D), Botulinum toxin serotype E (BoNT/E), Botulinum toxin serotype F (BoNT/F), Botulinum toxin serotype G (BoNT/G), Botulinum toxin serotype H (BoNT/H), Botulinum toxin serotype X (BoNT/X), and mosaic Botulinum toxins and/or subtypes and variants thereof. "Botulinum toxin", as used herein, also encompasses a "modified botulinum toxin". Further "botulinum toxin" as used herein also encompasses a botulinum toxin complex, (for example, the 300, 600 and 900kDa complexes), as well as the neurotoxic component of the botulinum toxin (150 kDa) that is unassociated with the complex proteins.

"Clostridial derivative" refers to a molecule which contains any part of a clostridial toxin. As used herein, the term "clostridial derivative" encompasses native or recombinant neurotoxins, recombinant modified toxins, fragments thereof, a Targeted vesicular Exocytosis Modulator (TEM), or combinations thereof.

"Clostridial toxin" refers to any toxin produced by a Clostridial toxin strain that can execute the overall cellular mechanism whereby a Clostridial toxin intoxicates a cell and encompasses the binding of a Clostridial toxin to a low or high affinity Clostridial toxin receptor, the internalization of the toxin/receptor complex, the translocation of the Clostridial toxin light chain into the cytoplasm and the enzymatic modification of a Clostridial toxin substrate. Non-limiting examples of Clostridial toxins include a Botulinum toxin like BoNT/A, a BoNT/B, a BoNT/C₁, a BoNT/D, a BoNT/E, a BoNT/F, a BoNT/G, a Tetanus toxin (TeNT), a Baratii toxin (BaNT), and a Butyricum toxin (BuNT). The BoNT/C₂ cytotoxin and BoNT/C₃ cytotoxin, not being neurotoxins, are excluded from the term "Clostridial toxin." A Clostridial toxin disclosed herein includes, without limitation, naturally occurring Clostridial toxin variants, such as, *e.g.*, Clostridial toxin isoforms and Clostridial toxin subtypes; non-naturally occurring Clostridial toxin variants, such as, *e.g.*, conservative Clostridial toxin variants, non-conservative Clostridial toxin variants, Clostridial toxin chimeric variants and active Clostridial toxin fragments thereof, or any combination thereof. A Clostridial toxin disclosed herein also includes a Clostridial toxin complex. As used herein, the term "Clostridial toxin complex" refers to a complex comprising a Clostridial toxin and non-toxin associated proteins (NAPs), such as, *e.g.*, a Botulinum toxin complex, a Tetanus toxin complex, a Baratii toxin complex, and a Butyricum toxin complex. Non-limiting examples of Clostridial toxin complexes include those produced by a *Clostridium botulinum*, such as, *e.g.*, a 900-kDa BoNT/A complex, a 500-kDa BoNT/A complex, a 300-kDa BoNT/A complex, a 500-kDa BoNT/B complex, a 500-kDa BoNT/C₁ complex, a 500-kDa BoNT/D complex, a 300-kDa BoNT/D complex, a 300-kDa BoNT/E complex, and a 300-kDa BoNT/F complex.

"Effective amount" as applied to the biologically active ingredient means that amount of the ingredient which is generally sufficient to induce a desired change in the subject.

"Implant" means a controlled release (*e.g*., pulsatile or continuous) composition or drug delivery system. The implant can be, for example, injected, inserted or implanted into a human body.

"Local administration" means administration of a pharmaceutical agent to or to the vicinity of a muscle or a subdermal location in a patient by a non-systemic route. Thus, local administration excludes systemic routes of administration, such as intravenous or oral administration.

Masseter Muscle Hypertrophy (MMH), used herein interchangeably as Masseter Muscle Prominence (MMP), refers to a condition wherein one or both masseter muscles are enlarged.

"Modified botulinum toxin" means a botulinum toxin that has had at least one of its amino acids deleted, modified, or replaced, as compared to a native botulinum toxin. Additionally, the modified botulinum toxin can be a recombinantly produced neurotoxin, or a derivative or fragment of a recombinantly made neurotoxin. A modified botulinum toxin retains at least one biological activity of the native botulinum toxin, such as, the ability to bind to a botulinum toxin receptor, or the ability to inhibit neurotransmitter release from a neuron. One example of a modified botulinum toxin is a botulinum toxin that has a light chain from one botulinum toxin serotype (such as serotype A), and a heavy chain from a different botulinum toxin serotype (such as serotype B). Another example of a modified botulinum toxin is a botulinum toxin coupled to a neurotransmitter, such as substance P.

"Mutation" means a structural modification of a naturally occurring protein or nucleic acid sequence. For example, in the case of nucleic acid mutations, a mutation can be a deletion, addition or substitution of one or more nucleotides in the DNA sequence. In the case of a protein sequence mutation, the mutation can be a deletion, addition or substitution of one or more amino acids in a protein sequence. For example, a specific amino acid comprising a protein sequence can be substituted for another amino acid, for example, an amino acid selected from a group which includes the amino acids alanine, asparagine, cysteine, aspartic acid, glutamic acid, phenylalanine, glycine, histidine, isoleucine, lysine, leucine, methionine, proline, glutamine, arginine, serine, threonine, valine, tryptophan, tyrosine or any other natural or non-naturally occurring amino acid or chemically modified amino acids. Mutations to a protein sequence can be the result of mutations to DNA sequences that when transcribed, and the resulting mRNA translated, produce the mutated protein sequence. Mutations to a protein sequence can also be created by fusing a peptide sequence containing the desired mutation to a desired protein sequence.

"Peripheral administration" means administration to a location away from a symptomatic location, as opposed to a local administration.

"Pharmaceutical composition" means a composition comprising an active pharmaceutical ingredient, such as, for example, a clostridial toxin active ingredient such as a botulinum toxin, and at least one additional ingredient, such as, for example, a stabilizer or excipient or the like. A pharmaceutical composition is therefore a formulation which is suitable for diagnostic or therapeutic administration to a subject, such as a human patient. The pharmaceutical composition can be, for example, in a lyophilized or vacuum dried condition, a solution formed after reconstitution of the lyophilized or vacuum dried pharmaceutical composition, or as a solution or solid which does not require reconstitution.

"Pharmacologically acceptable excipient" is synonymous with "pharmacological excipient" or "excipient" and refers to any excipient that has substantially no long term or permanent detrimental effect when administered to mammal and encompasses compounds such as, *e.g.*, stabilizing agent, a bulking agent, a cryo-protectant, a lyo-protectant, an additive, a vehicle, a carrier, a diluent, or an auxiliary. An excipient generally is mixed with an active ingredient or permitted to dilute or enclose the active ingredient and can be a solid, semi-solid, or liquid agent. It is also envisioned that a pharmaceutical composition comprising a Clostridial toxin active ingredient can include one or more pharmaceutically acceptable excipients that facilitate processing of an active ingredient into pharmaceutically acceptable compositions. Insofar as any pharmacologically acceptable excipient is not incompatible with the Clostridial toxin active ingredient, its use in pharmaceutically acceptable compositions is contemplated. Non-limiting examples of pharmacologically acceptable excipients can be found in, *e.g.*, Pharmaceutical Dosage Forms and Drug Delivery Systems (Howard C. Ansel et al., eds., Lippincott Williams & Wilkins Publishers, 7th ed. 1999); Remington: The Science and Practice of Pharmacy (Alfonso R. Gennaro ed., Lippincott, Williams & Wilkins, 20th ed. 2000); Goodman & Gilman's The Pharmacological Basis of Therapeutics (Joel G. Hardman et al., eds., McGraw-Hill Professional, 10th ed. 2001); and Handbook of Pharmaceutical Excipients (Raymond C. Rowe et al., APhA Publications, 4th edition 2003), each of which is hereby incorporated by reference in its entirety.

"TEM" as used herein, is synonymous with "Targeted Exocytosis Modulator" or "retargeted endopeptidase." Generally, a TEM comprises an enzymatic domain from a Clostridial toxin light chain, a translocation domain from a Clostridial toxin heavy chain, and a targeting domain. The targeting domain of a TEM provides an altered cell targeting capability that targets the molecule to a receptor other than the native Clostridial toxin receptor utilized by a naturally-occurring Clostridial toxin. This re-targeted capability is achieved by replacing the naturally-occurring binding domain of a Clostridial toxin with a targeting domain having a binding activity for a non-Clostridial toxin receptor. Although binding to a non-Clostridial toxin receptor, a TEM undergoes all the other steps of the intoxication process including internalization of the TEM/receptor complex into the cytoplasm, formation of the pore in the vesicle membrane and di-chain molecule, translocation of the enzymatic domain into the cytoplasm, and exerting a proteolytic effect on a component of the SNARE complex of the target cell.

"Treating" or "treatment" means to alleviate (or to eliminate) at least one symptom, either temporarily or permanently.

"Therapeutically effective amount" refers to an amount sufficient to achieve a desired therapeutic or cosmetic effect.

"Variant" means a Clostridial neurotoxin, such as wild-type botulinum toxin serotype A, B, C, D, E, F or G, that has been modified by the replacement, modification, addition or deletion of at least one amino acid relative to wild-type botulinum toxin, which is recognized by a target cell, internalized by the target cell, and catalytically cleaves a SNARE (SNAP (Soluble NSF Attachment Protein) Receptor) protein in the target cell.

An example of a variant neurotoxin component can comprise a variant light chain of a botulinum toxin having one or more amino acids substituted, modified, deleted and/or added. This variant light chain may have the same or better ability to prevent exocytosis, for example, the release of neurotransmitter vesicles. Additionally, the biological effect of a variant may be decreased compared to the parent chemical entity. For example, a variant light chain of a botulinum toxin type A having an amino acid sequence removed may have a shorter biological persistence than that of the parent (or native) botulinum toxin type A light chain.

### METHODS OF TREATMENT

In a first aspect, a method for treating MMH is provided. The present method comprises administering a therapeutically effective amount of a clostridial derivative into a muscle, such as the masseter muscle, to reduce muscle activity. This reduced muscle activity results in a reduction in the size of the muscle, which is perceived as facial shaping or slimming.

Other aspects to be described include a method to reduce lower face width in the masseter muscle region, a method to temporarily reduce lower face convexity or width associated with masseter muscle prominence, a method to alter the contour of a lower face of a human, a method to sculpt or reshape a lower face of a human in the masseter muscle region, and a method to reduce lower face convexity associated with the masseter muscle. In another aspect, a method to reduce face asymmetry is provided. These methods comprise, in one embodiment, locally administering a Clostridial derivative, such as a botulinum toxin. In other embodiments, the methods comprise identifying a region of maximal bulge of the masseter muscle and injecting a Clostridial derivative at a plurality of injection sites in the region to administer a dose of Clostridial derivative. In still other embodiments, the methods comprise identifying a line that extends from the lateral commissure of the mouth to the point where the ear lobe attaches to the face; identifying a region of maximal bulge of the masseter muscle with the jaw in a clenched position; determining a treatment area that (i) includes the region of maximal bulge, (ii) is positioned at or below the line, (iii) is posterior to the risorius muscle and (iv) is anterior to the parotid gland; and administering a Clostridial derivative at a plurality of administration sites in the treatment area to administer a dose of Clostridial derivative. The method can be performed unilaterally or bilaterally.

For the methods, a therapeutically effective amount of a Clostridial derivative is administered to a treatment area within the masseter muscle of a subject. One approach to identifying the treatment area is to identify a region of maximal bulge of the masseter muscle. The region of maximal bulge corresponds to the treatment area, in this embodiment. The region of maximal bulge of the masseter muscle is identified for example by having the subject clench the jaw. Typically, the subject will maximally clench his/her jaw (mouth closed and teeth together) which allows the masseter muscle to be more visible. In addition to or rather than visual inspection for the maximal bulge of the masseter muscle, the maximal bulge can be identified or confirmed by manually palpating the masseter muscle to feel for the maximal bulge.

Another approach for identifying a treatment area is shown in **FIG. 1**. **FIG. 1** is a schematic of side view of a human face **10**. In this embodiment, a treatment area is determined by identifying a line that extends from the corner of the mouth, also referred to as the lateral commissure **12** of the mouth, to the point **14** where the ear lobe attaches to the face. This line is identified in FIG. 1 as **Line A**. A region of maximal bulge of the masseter muscle is identified and in one approach this is done by asking the subject to clench the jaw. Typically, the subject will maximally clench his/her jaw (mouth closed and teeth together) which allows the masseter muscle to be more visible. In addition to or rather than visual inspection for the maximal bulge of the masseter muscle, the maximal bulge can be identified or confirmed by manually palpating the masseter muscle to feel for the maximal bulge. A treatment area is defined that includes the region of maximal bulge, is positioned at or below the line (Line A of FIG. 1), is posterior to the risorius muscle **16** and is anterior to the parotid gland **18**. This treatment area is denoted in FIG. 1 by dashed circle region **20**.

The identified treatment area can be visually marked with imaginary lines or can be physically marked on the skin. In one embodiment, Line A is identified and is not physically marked but its position is visualized. In another embodiment, Line A is identified and is physically marked on the skin. The same approach may be applied to the risorius muscle, the maximal bulge, the parotid gland, and the treatment area, in that each can be visually marked with imaginary lines or can be physically marked on the skin.

Once a treatment area is identified, the Clostridial derivative, e.g., botulinum toxin, is administered. In one embodiment, the Clostridial derivative is administered at a single administration site in the treatment area and in another embodiment, it is administered at a plurality of administration sites in the treatment area. The plurality of administration sites may range from 2-10, 2-9, 2-8, 2-7, 2-6, 2-5, 2-4, 2-3, 3-10, 3-9, 3-8, 3-7, 3-6, 3-5, or 3-4. In one embodiment, the number of administration sites in the plurality is 2, 3, 4, 5, 6, 7, 8, 9, or 10. The administration sites are spaced from one another equally or unequally. In one embodiment, at least one site in the plurality of administration sites is spaced from an adjacent site in the plurality of administration sites by about 0.5 cm, 0.75 cm, or 1 cm. In another embodiment, each administration site in the plurality of administration sites is spaced from an adjacent administration site by about 0.5 cm, 0.75 cm, or 1 cm. In another embodiment, each administration site in the plurality of administration sites is spaced from any adjacent administration site by about 0.5 cm, 0.75 cm, or 1 cm. When the Clostridial derivative is administered by injection, the administration sites are referred to as injection sites.

The pattern or arrangement of administration sites, particularly when there is more than one, can vary. For example, in one embodiment, the most bulky point of the masseter muscle or the maximal bulge of the masseter muscle is identified and is assigned as the first administration site; and subsequent or further administration sites are placed relative to the first administration site. The plurality of administration sites can form a triangle or an inverted triangle in the treatment area. The plurality of administration sites can form a line, where in one embodiment, the administration sites are along a line that extends directionally with the jaw line. In another embodiment, the administration sites are along a line that extends directionally from the shoulder to the eyelid. The administration sites, in one embodiment, are evenly spaced to fill the treatment area.

It will be appreciated that administration can be unilateral (to one masseter muscle) or bilateral (to the masseter muscle on each side of the face).

The Clostridial derivative can be administered by injection, transdermally, topically, or by implantation. Exemplary implantation includes implanting a slow-release device, such as a polymeric implant or mini-osmotic pump, that releases the Clostridial derivative. When administered by injection, the injection can be intramuscular, non-intramuscular, intradermal, or subcutaneous. In one embodiment, the position of needle for injection is perpendicular to the full depth of the masseter muscle. That is, the needle direction within the muscle bulk is perpendicular and not oblique, and the injection volume is distributed within the deeper and more superficial muscle layers. Without being limited to theory, administration by injection with a needle directed to be perpendicular to the muscle bulk helps ensure toxin delivery to the masseter muscle. In another embodiment, the position of the needle is selected to distribute the dose to be administered at a given injection site into the superficial muscle and/or tissue and into deeper muscle layers. Without being limited to theory, distribution of the dose into superficial muscle and/or tissue and into deeper muscle layers provides the benefit of uniform toxin distribution throughout the various muscle layers. In other embodiments, the Clostridial derivative is administered with the jaw in a relaxed position.

In one embodiment, each masseter muscle is treated by administering a dose of botulinum toxin at a plurality of administration sites in each muscle, where each injection site in the plurality is spaced from an adjacent injection site by at least about 0.25 cm, 0.30 cm, 0.35 cm, 0.40 cm, 0.45 cm, 0.50 cm, 0.55 cm, 0.60 cm, 0.65 cm, 0.70 cm, 0.75 cm, 0.80 cm, 0.85 cm, 0.90 cm, 0.95 cm, 1.0 cm, 1.25 cm, 1.50 cm or 2.0 cm. In another embodiment, each injection site in the plurality is spaced from an adjacent injection site by between about 0.25-1.5 cm, 0.25-1.10 cm, 0.35-1.5 cm, 0.35-1.10 cm, 0.45-1.5 cm, 0.45-1.10 cm, 0.50-1.50 cm, 0.50-1.10 cm, 0.75-1.5 cm, 0.75-1.10 cm, 0.80-1.5 cm, or 0.90-1.10 cm. In one embodiment, each injection site is spaced apart from an adjacent site by about 1cm. Without being limited to theory, an optimal spacing provides the benefits of uniform and contiguous distribution of the total dose across the entire treatment area to produce the intended result. When an injection site is spaced too distantly from adjacent injection sites, the dose administered at each injection site may affect different portions of the muscle, resulting in an uneven toxin distribution across the treatment area and creating lumpy or bulgy appearance. Too closely spaced injection sites also create uneven toxin distribution across the treatment area.

The methods comprise, in some embodiments, assessing masseter muscle prominence or reviewing an assessment of masseter muscle prominence. There are a variety of techniques to assess masseter muscle prominence including, for example, computed tomography (CT), cone beam computed tomography (CBCT), magnetic resonance imaging (MRI), ultrasound, questionnaires, three-dimensional quantitative analysis of facial morphology based on, for example, an image subtraction technique, moiré topography, liquid crystal scanning, light luminance scanning, laser scanning, stereo-lithography, or passive stereophotogrammetry. By way of example, for the study described in Example 1, lower facial volume was measured using a three-dimensional imaging system and CT.

Other techniques for assessing masseter muscle prominence including a lower facial width measure where lower facial width, lines A, B, and C are drawn from on a frontal facial image. Line A corresponds to the width of the face at the level of the stomion, the midline point at the junction of the upper and lower lip vermillion. Line B corresponds to the alar base width, defined as the distance between the left and right alar rim, and not the left and right alar crease junction. Line C corresponds to the distance between the right and left medial canthus. Intercanthal distance (C) and alar base width (B) are anticipated to remain constant during the course of the study and to be unaffected by study treatment. The width of the face (A) as well as ratio of alar base width to facial width (B/A) and the ratio of intercanthal width to facial width (C/A) are calculated for the subject. Another technique is the mandibular angle, which can be calculated by averaging the left and right side angle and rounding up to an integer. The mandibular facial angle is the angle between (1) an oblique line 1 drawn across the contour of the horizontal part of the jawline and another oblique line 2 drawn from the most lateral aspect of cheek to the angle of the mandible outlining the plane of the face containing the vertical ramus of the mandible. Lines 1 and 2 intersect at the apex of the angle of the mandible, and the mandibular angle is taken as the internal angle between lines 1 and 2.

In a preferred embodiment, masseter muscle prominence before, during or after treatment is assessed using a questionnaire. The questionnaire may be one that is completed by the subject seeking treatment or being treated or by a third party, such as the individual administering the treatment or the assessment. Exemplary questionnaires include the Lower Facial Shape Classification (LFSC); the Lower Facial Shape Questionnaire (LFSQ); or the Masseter Muscle Prominence Scale (MMPS).

The LFSC is a 4-point scale designed to provide an investigator's or a subject's assessment of a subject's lower facial shape. The scale and the lower facial shape descriptors associated with each grade are set forth in Table 1.

**Table 1: Lower Facial Shape Classification (LFSC)**

| LFSC Grade | LFSC Descriptor |
|---|---|
| 1 | Lower facial shape is oval. |
| | Front view: narrow, curved jaw contour |
| 2 | Lower facial shape is round. |
| | Front view: wide, curved jaw contour |
| 3 | Lower facial shape is square. |
| | Front view: wide, square jaw contour |
| 4 | Lower facial shape is trapezoid. |
| | Front view: very wide, protruding jaw contour |

The LFSQ seeks to obtain (1) symptom assessment, (2) impact assessment; (3) satisfaction assessment, or (4) combinations thereof. The questionnaire aims to assess the subject's view on the efficacy as well as any adverse effects which may be due to the treatment. The questionnaire presents seven questions for a subject to consider and respond to about his/her lower face. When answering questions, the responder is asked to consider only his/her lower face - that is, the bottom half of the face from the top of cheeks to the chin**.** **FIGS. 7A-7B** present the questions on the LFSQ.

The MMPS provides an approach to assess masseter muscle prominence during rest and maximum contraction (clenched teeth with full force) by separately evaluating the right and left side of the subject's face using a rating scale of 1-5. In this assessment, masseter muscle prominence is ascertained by visually inspecting the masseter muscle, palpating the masseter muscle, or both. Visual inspecting comprises inspecting the contour of the face of the subject in the clenched and unclenched state, defining the posterior and anterior border of each masseter and the superior and inferior margins, rating the prominence of the masseter muscle using a scale, such as that shown in Table 2. Inspecting by palpating comprises feeling the dimensions and surface texture of each masseter while the subject clenches and relaxes, confirming the posterior and anterior border of each masseter and the superior and inferior margins, distinguishing the master muscle from the bone and other non-muscular soft tissue (e.g. fat) while the subject clenches and relaxes, rating the prominence of the masseter muscle using a scale, such that shown in Table 2.

**Table 2: Masseter Muscle Prominence Scale (MMPS)**

| **Rating Grade** | **Clinical Evaluation** |
|---|---|
| Minimal (1) | AT REST: With mouth closed and no clenching, surface overlying masseter is concave. There is no contour contributed by masseter muscle. Masseter is not palpable. |
| | AT CLENCH: No visible difference in contour compared with when mouth is closed, no clenching. Masseter is minimally palpable and difficult to define. |
| Mild (2) | AT REST: With mouth closed and no clenching, surface overlying masseter is flat or slightly concave. The contour contributed by masseter muscle may or may not be visible. Masseter is minimally palpable. |
| | AT CLENCH: With clenching, minimal difference in lower facial contour compared with when mouth is closed, no clenching. Portion of masseter bulk may be visible and palpable. |
| Moderate (3) | AT REST: With mouth closed and no clenching, surface overlying masseter is flat or convex. The contour contributed by masseter muscle may or may not be visible. Masseter is palpable. |
| | AT CLENCH: With clenching, the lower face is more convex in contour compared with when mouth is closed, no clenching. Masseter bulk is easily identifiable, palpable, and firm. |
| Marked (4) | AT REST: With mouth closed and no clenching, surface overlying masseter is convex. The masseter muscle, in conjunction with the chin and jawline, creates a square lower facial contour. Masseter is palpable and firm. |
| | AT CLENCH: With clenching, the lower face is wider and squarer compared with when mouth is closed, no clenching. Masseter is palpable and firm or hard. |
| Very Marked (5) | AT REST: With mouth closed and no clenching, surface overlying masseter is convex. The masseter muscle, in conjunction with the chin and jawline, creates a trapezoidal lower facial contour. Masseter is palpable and firm. |
| | AT CLENCH: With clenching, the lower face is more trapezoidal compared with when mouth is closed, no clenching. Masseter is palpable and hard. |

In some embodiments, the MMPS is used to select subjects with a square face for treatment with a Clostridial derivative. A square face can result from bone origin hypertrophy or soft tissue origin hypertrophy, including muscle hypertrophy, parotid gland enlargement and fat deposition, or a combination of both. Bone origin hypertrophy shows a square face and a prominent mandibular angle. Bone origin hypertrophy is not treatable by Clostridial derivative administration. In some embodiments, the MMPS is used to select subjects having MMH from those having bone origin hypertrophy.

Studies were conducted as described in Example 1 using the MMPS to identify subjects with marked (Grade 4) or very marked (Grade 5) for treatment with a Clostridial derivative. For purposes of the study, and to verify the MMPS, lower facial volume of the subjects was also measured using an imaging method (see Materials and Methods below). Botulinum toxin was used as the exemplary Clostridial derivative, and it will be appreciated that other Clostridial derivatives, described infra, would be suitable for the methods. In the study of Example 1, subjects with marked or very marked MMH, according to the MMPS, were identified for treatment with a total bilateral dose of botulinum toxin of 24 units, 48 units, 72 units or 96 units (see Table 1-1 in Example 1). The subjects were 18 years of age or older. The dose was evenly divided into two portions for bilateral, intramuscular injection into each masseter muscle. Three injections were made into each masseter muscle, for a total of six injections for bilateral treatment. For example, each subject in cohort 1 was treated with total dose of 24 units, the total dose divided equally for bilateral treatment with 12 units per masseter muscle. The 12 unit dose per masseter muscle was divided equally into 3 injection sites per masseter muscle. Each subject in cohort 4 was treated with total dose of 96 units, the total dose divided equally for bilateral treatment with 48 units per masseter muscle. The 48 unit dose per masseter muscle was divided equally into 3 injection sites per masseter muscle. The 3 injection sites in each masseter muscle were within a treatment area at or below a line that extended from the lateral commissure of the mouth to the point where the ear lobe attaches to the face, that included the region of maximal bulge of the masseter muscle, and posterior to the risorius muscle and anterior to the parotid gland.

Each subject was evaluated monthly after treatment with the indicated dose for a one-year period. At each monthly visit lower facial volume (cm³) was calculated using a three-dimensional imaging system and the MMPS was completed. With reference to **FIGS. 2A-2B**, the change in lower face volume 90 days after treatment (FIG. 2A) and monthly over the 180 day evaluation period (FIG. 2B) is shown**.** **FIG. 2A** is a bar graph showing the least squares mean (LSMean) change in lower face volume (in cm³) 90 days after treatment with the indicated dose of botulinum toxin in the masseter muscle, the volume change at day 90 day relative to lower face volume prior to treatment (baseline), where lower face volume is calculated using a three-dimensional imaging system. There was a significant reduction in facial volume at day 90 compared to the placebo group, with the reduction in facial volume in the group dose with 48 units statistically significantly more compared to the group treated with 24 units**.** **FIG. 2B** shows the change in facial volume at each monthly evaluation over the one-year post treatment period. The least squares mean (LSMean) change in lower face volume (in cm³) was reduced for at least six months after treatment (p ≤ 0.05) in all treatment groups (24 units (circles), 48 units (squares), 72 units (triangles), and 96 units (diamonds). The changes in lower face volume assessed by the three-dimensional imaging VECTRA were confirmed by CT evaluations.

The study subjects of Example 1 completed the MMPS at the monthly evaluations. Prior to treatment, study subjects were evaluated using the MMPS and had a Grade 4 (marked) or Grade 5 (very marked) masseter muscle prominence**.** **FIG. 3A** shows the percent of subjects in each treatment dose cohort that achieved a rating of Grade 3 or less according to a clinician's evaluation using the MMPS for the subject**.** **FIG. 3B** shows the percent of subjects in each treatment dose cohort that achieved a change in rating of 2 or more according to a clinician's evaluation using the MMPS. A statistically significant response was observed through post treatment day 180 for subjects treated with a total bilateral botulinum toxin dose of 48 units (squares), 72 units (triangles), and 96 units (diamonds). Notably, the total bilateral botulinum toxin dose of 72 units (36 unit unilateral dose) achieved at day 120 a greater number of subjects with an MMPS of Grade 3 or less (FIG. 3A) and also a greater number of subjects with a two or greater MMPS change in grade (FIG. 3B) than the subjects treated with the higher 96 unit bilateral dose (48 units unilaterally).

With reference to **FIGS. 4A-4B**, the MMPS evaluations at post treatment day 90 are shown. In **FIG. 4A**, the percent of subjects in each treatment dose cohort that achieved a rating of Grade 3 or less according to the MMPS 90 days after treatment with a bilateral botulinum toxin dose at the noted dosages is shown. In **FIG. 4B** the percent of subjects in each treatment dose cohort that achieved a change in rating of 2 or more according to the MMPS 90 days after treatment with a bilateral botulinum toxin dose at the indicated doses is shown. The difference in response provided by the 48 unit bilateral dose and the 72 unit bilateral dose was statistically significant for the responders with a greater than or equal to 2 change in MMPS ranking.

The enhanced effect provided by a bilateral treatment dose of greater than 48 units (24 units unilaterally) and less than about 96 units (48 units unilaterally) is seen in **FIG. 5**. FIG. 5 is a bar graph showing the duration of effect in the subjects of each treatment dose cohort that achieved a change in rating of 2 or more according to the MMPS after treatment with a bilateral botulinum toxin dose of 24 units, 48 units, 72 units, and 96 units. The subjects treated with the 72 unit bilateral dose (36 unit unilateral dose) had a longer duration of effect than the 48 unit bilateral dose and the 96 unit bilateral dose.

As described in Example 2, the subjects enrolled in the study of Example 1 were eligible for a second treatment (retreatment) if based on a clinician's assessment using the MMPS at day 180 (180 days after in first dose at the indicated dosages in Example 1) the subject had marked (Grade 4) or very marked (Grade 5) bilateral masseter muscle hypertrophy. For the subjects eligible for retreatment, the second treatment was given at the day 180 visit at the same dose level and same treatment location as at the beginning (day 1) of the study. The subjects that were retreated were evaluated at monthly intervals for 180 days to determine lower facial volume (cm³) using a three-dimensional imaging system. Results are shown in FIG. 6.

The least squares mean (LSMean) change in lower face volume (in cm³) as a function of time after treatment with botulinum toxin in the masseter muscle is shown in FIG. 6. The arrow TX1 indicates a first treatment with botulinum toxin (Example 1) and the arrow at TX2 indicates the second treatment with botulinum toxin (Example 2). The second treatment markedly reduced lower face volume in this patient group.

The studies of Examples 1 and 2 demonstrate that administration of a Clostridial derivative, unilaterally or bilaterally, to subjects with MMH, reduces lower face volume, relative to MMH subjects left untreated, for at least about 90 days or at least about 180 days. Treatment of MMH with the Clostridial derivative botulinum toxin provided a non-permanent, temporary reduction in lower face volume, thereby reducing lower face width in the masseter muscle region, reducing prominence of the masseter muscle, and temporarily reducing lower face convexity or width. In this regard, "temporarily" intends non-permanent, and in one embodiment is at least about 60 days, at least about 90 days, at least about 120 days, or at least about 180 days, or at least about 270 days, or at least about 360 days. The study in Example 2 demonstrated that a second treatment provided a volume reduction in the subjects with marked or very marked masseter muscle prominence according to the MMPS, where a bilateral dose greater than 48 units and equal to or less than 96 units providing an at least 2-grade improvement in MMH severity when evaluated using the MMPS. From this study, the subjects treated with a first bilateral dose of 72 units enjoyed a longer duration and more pronounced effect that subjects treated with a bilateral dose of 96 units.

The studies in Examples 1 and 2 also demonstrate that the treatments in accord with the methods described herein do not appear to have any impact on the dentition and/or bone density of the subjects as assessed by CT scans and dental examinations.

As previously mentioned, the studies in Examples 1 and 2 were conducted using botulinum toxin as a model Clostridial derivative. It will be appreciated that other Clostridial derivatives are contemplated and suitable. In some embodiments, the clostridial derivative usable with the present methods includes a native, recombinant clostridial toxin, recombinant modified toxin, fragments thereof, TEMs, or combinations thereof. An example of a Clostridial derivative is a botulinum toxin. Botulinum neurotoxins (BoNTs) such as, for example, BoNT/A, BoNT/B, etc., act on the nervous system by blocking the release of neurosecretory substances such as neurotransmitters. The action of BoNT is initiated by its binding to a receptor molecule on the cell surface, then the toxin-receptor complex undergoes endocytosis. Once inside the cell, BoNT cleaves exocytotic specific proteins responsible for neurotransmitter docking and release from the cell known as the SNARE proteins (soluble N-ethylmaleimide-sensitive factor attachment protein receptor). The resulting transient chemodenervation has been utilized medically to block motor neurotransmission at the neuromuscular junction leading to a variety of therapeutic applications.

The botulinum toxin can be a botulinum toxin type A, type B, type C₁, type D, type E, type F, or type G, type H, type X, and mosaic Botulinum toxins and/or subtypes and variants thereof. The botulinum neurotoxin can be a recombinantly made botulinum neurotoxins, such as botulinum toxins produced by *E. coli*. In alternative embodiments, the clostridial derivative is a TEM. The botulinum neurotoxin can be a modified neurotoxin; that is, a botulinum neurotoxin which has at least one of its amino acids deleted, modified or replaced, as compared to a native toxin, or the modified botulinum neurotoxin can be a recombinant produced botulinum neurotoxin or a derivative or fragment thereof. In certain embodiments, the modified toxin has an altered cell targeting capability for a neuronal or non-neuronal cell of interest. This altered capability is achieved by replacing the naturally-occurring targeting domain of a botulinum toxin with a targeting domain showing a selective binding activity for a non- botulinum toxin receptor present in a non- botulinum toxin target cell. Such modifications to a targeting domain result in a modified toxin that is able to selectively bind to a non-botulinum toxin receptor (target receptor) present on a non-botulinum toxin target cell (re-targeted). A modified botulinum toxin with a targeting activity for a non-botulinum toxin target cell can bind to a receptor present on the non-botulinum toxin target cell, translocate into the cytoplasm, and exert its proteolytic effect on the SNARE complex of the target cell. In essence, a botulinum toxin light chain comprising an enzymatic domain is intracellularly delivered to any desired cell by selecting the appropriate targeting domain. Further "botulinum toxin" as used herein also encompasses a botulinum toxin complex, (for example, the 300, 600 and 900kDa complexes), as well as the neurotoxic component of the botulinum toxin (150 kDa) that is unassociated with the complex proteins.

The Clostridial derivative, such as a botulinum toxin, can be stored in lyophilized, vacuum dried form in containers under vacuum pressure or as stable liquids. Prior to lyophilization the botulinum toxin can be combined with pharmaceutically acceptable excipients, stabilizers and/or carriers, such as, for example, albumin, or the like. Acceptable excipients or stabilizers include protein excipients, such as albumin or gelatin, or the like, or non- protein excipients, including poloxamers, saccharides, polyethylene glycol, or the like. In embodiments containing albumin, the albumin can be, for example, human serum albumin or recombinant human albumin, or the like. The lyophilized material can be reconstituted with a suitable liquid such as, for example, saline, water, or the like to create a solution or composition containing the botulinum toxin to be administered to the patient.

In some embodiments, the Clostridial derivative is provided in a controlled release system comprising a polymeric matrix encapsulating the Clostridial derivative, wherein fractional amount of the clostridial derivative is released from the polymeric matrix over a prolonged period of time in a controlled manner. Controlled release neurotoxin systems have been disclosed for example in U.S. patents 6,585,993; 6,585,993; 6,306,423 and 6,312,708, each of which is hereby incorporated by reference in its entirety.

The therapeutically effective amount of the Clostridial derivative, for example a botulinum toxin, administered according to the present methods can vary according to the potency of the toxin and the extent of the masseter muscle hypertrophy, including its prominence and other various patient variables including size, weight, age, and responsiveness to therapy. The potency of the botulinum toxin is expressed as a multiple of the LD₅₀ value for the mouse, one unit (U) of toxin being defined as being the equivalent amount of toxin that kills 50% of a group of 18 to 20 female Swiss-Webster mice, weighing about 20 grams each.

The therapeutically effective amount of the botulinum toxin, in the present methods can vary according to the potency of a particular botulinum toxin, as commercially available Botulinum toxin formulations do not have equivalent potency units. For example, one unit of BOTOX^{®} (onabotulinumA), a botulinum toxin type A available from Allergan, Inc., has been reported to have a potency unit that is approximately equal to 3 to 5 units of DYSPORT^{®} (abobotulinumA), also a botulinum toxin type A available from Ipsen Pharmaceuticals. MYOBLOC^{®}, a botulinum toxin type B available from Elan, has a much lower potency unit relative to BOTOX^{®}. In some embodiments, the botulinum neurotoxin can be a pure toxin, devoid of complexing proteins, such as XEOMIN^{®} (incobotulinumtoxinA). One unit of IncobotulinumtoxinA has been reported to have a potency approximately equivalent to one unit of onabotulinumtoxinA. Thus, the quantity of toxin administered, and the frequency of its administration will be at the discretion of the physician responsible for the treatment and will be commensurate with questions of safety and the effects produced by a particular toxin formulation.

The dosages used in the methods disclosed herein range from about 0.01 to about 1,000 units; for example, up to about 500 units, and preferably in the range from about 10 to about 460 units per patient per treatment. More particularly, and in accord with the findings of Example 1 and Example 2, a therapeutically effective amount of a botulinum toxin type A, such as BOTOX^{®}, is administered to the masseter muscle. The therapeutically effective amount ranges, in some embodiments, from about 1 unit/masseter muscle to about 200 units/masseter muscle. In other embodiments, the therapeutically effective amount administered to each masseter muscle is from about 5-100 units, 5-50 units, or 10-50 units. In one embodiment, the dose administered to each masseter muscle is greater than or equal to 24 units and less than or equal to 48 units, and in a particular embodiment the dose administered to each masseter muscle is 36 units.

In other embodiments, the dose for treatment that is administered to each masseter muscle is 1 unit, 2 units, 3 units, 4 units, 5 units, 6 units, 7 units, 8 units, 9 units, 10 units, 11 unit, 12 units, 13 units, 14 units, 15 units, 16 units, 17 units, 18 units, 19 units, 20 units, 21 units, 22 units, 23 units, 24 units, 25 units, 26 units, 27 units, 28 units, 29 units, 30 units, 31 units, 32 units, 33 units, 34 units, 35 units, 36 units, 37 units, 38 units, 39 units, 40 units, 41 units, 42 units, 43 units, 44 units, 45 units, 46 units, 47 units, 48 units, 49 units, 50 units, 51 units, 52 units, 53 units, 54 units, 55 units, 56 units, 57 units, 58 units, 59 units, 60 units, 61 unit, 62 units, 63 units, 64 units, 65 units, 66 units, 67 units, 68 units, 69 units, 70 units, 71 units, 72 units, 73 units, 74 units, 375 units, 76 units, 77 units, 78 units, 79 units, 80 units, 81 units, 82 units, 83 units, 84 units, 85 units, 86 units, 87 units, 88 units, 89 units, 90 units, 91 units, 92 units, 93 units, 94 units, 95 units, 96 units, 97 units, 98 units, 99 units, 100 units, 101 units, 102 units, 103 units, 104 units, 105 units, 106 units, 107 units, 108 units, 109 units or 110 units.

The volume in which a dose to be administered will vary according to the formulation. A typical volume range is between 0.1-5 mL per treatment area, more particularly between 0.1-4 mL, 0.2-4 mL, 0.2-2 mL, 0.3-4 mL, 0.3-2 mL, 0.3-1.5 mL and 0.3-1.2 mL. In one embodiment, the volume per injection is between about 0.05-1 mL, 0.05-0.8 mL, 0.05-0.6 mL, 0.1-2 mL, 0.1-1.5 mL, 0.1-1.0 mL, 0.1-0.8 mL, 0.1-0.7 mL, 0.1-0.6 mL, 0.1-0.5 mL, and 0.1-0.4 mL.

It will be appreciated that a subject treated in accord with the methods described herein can be assessed after treatment to ascertain treatment efficacy and safety. The therapeutically effective amount or dose of the Clostridial derivative can be modified depending on the assessment of efficacy and safety. In one embodiment, the assessment of efficacy corresponds to a change in the masseter muscle prominence rating using MMPS. In some embodiments, the assessment of safety includes assessing of adverse effects such as mastication disorder, injection site pain, facial paresis, headache. In some embodiments, safety assessment further includes determining whether the treatment methods described herein has any impact on the mandible and/or dentition of a subject. In some embodiments, the impact on the mandible bone is assessed by CT. In some other embodiments, the impact on dentition is assessed by dental examination.

In some embodiments, the assessing step is carried out from about 1 month to about 6 months after MMH treatment. In view of the change in muscle volume determined during the assessment step, repeated treatments of the MMH may be carried out. The change in muscle volume determined during the assessment step may be taken into account to determine a therapeutically effective amount of Clostridial derivative for subsequent treatments.

In some embodiments, the methods further comprise administering a second treatment to patients or subjects having marked (grade 4) or very marked (grade 5) bilateral masseter hypertrophy according to the MMPS. In an alternative embodiment, the present method comprises assessing a subject's lower facial shape using the LFSC as described herein, identifying patients or subjects having wide, angular jaw line contour (grade 3) or trapezoid with wider protruding jaw line contour (grade 4) as eligible for subsequent treatment.

Efficacy of MMH treatment in accord with the method may be assessed using the MMPS. In some embodiments, the present method comprises rating the masseter muscle prominence of a subject before and after treatment, identifying subjects having a MMPS grade reduction of 1 or greater from baseline grade, categorizing the subjects having a MMPS grade reduction of 1 or greater from baseline grade as positive responders to the treatment. In alternative embodiments, the present method comprises classifying the lower facial shape of a subject using the 4-point scale LFSC before and after MMH treatment, identifying subjects having a LFSC grade reduction of 1 or greater from baseline grade, categorizing the subjects having a LFSC grade reduction of 1 or greater from baseline grade as positive responders to the treatment.

In other embodiments, the present disclosure provides a method for treating MMH. In some embodiments, the method comprises using one or more of the measures described herein to select subjects eligible for MMH treatment, administering to the subject a therapeutically effective amount of a Clostridial derivative, assessing the efficacy of the MMH treatment using one or more of the efficacy measures described herein, and re-administering to the subject a second therapeutically effective amount of the Clostridial derivative. In one embodiment, the selecting step comprises rating the masseter muscle prominence of a subject using the MMPS. In some other embodiments, the selecting step further comprises classifying a subject's lower facial shape using the Lower Facial shape classification scale, obtaining the subject' own assessment of lower facial shape using the LFSQ, or combination thereof.

### KIT OF PARTS

In another aspect, a kit of parts is provided. In one embodiment a kit comprising a botulinum toxin and instructions for administration of botulinum toxin into a muscle for the treatment of masseter muscle hypertrophy, and optionally an MMPS scale and/or instructions using an MMPS to identify a subject for treatment.

In another aspect, a kit of parts is provided that comprises: a) botulinum toxin in an amount of about 5-100 Units, and b) instructions for administration of botulinum toxin into a muscle for the treatment of masseter muscle hypertrophy, in which administering botulinum toxin into the masseter muscle of a subject having masseter muscle hypertrophy. In one embodiment, the amount of botulinum toxin is between about 10-50 Units or between 20-100 Units, preferably between about 12-48 Units or 24-96 Units, more preferably between about 24-36 Units or 48-72 Units. In a specific embodiment, the amount of botulinum toxin is 12 Units, 24 Units, 36 Units, 48 Units, 72 Units or 96 Units. In one embodiment, the botulinum toxin is packaged in multiple portions.

In another aspect, a kit of parts is provided that comprises: a) botulinum toxin in an amount of about 5-100 Units, and b) instructions for administration of botulinum toxin into a muscle for reducing lower face width in a masseter muscle region, in which i) identifying a region of maximal bulge of the masseter muscle, and ii) injecting botulinum toxin at a plurality of injection sites in the region to administer a dose of botulinum toxin to reduce lower face width in the masseter muscle region. In one embodiment, the amount of botulinum toxin is between about 10-50 Units or between 20-100 Units, preferably between about 12-48 Units or 24-96 Units, more preferably between about 24-36 Units or 48-72 Units. In a specific embodiment, the amount of botulinum toxin is 12 Units, 24 Units, 36 Units, 48 Units, 72 Units or 96 Units. In one embodiment, the botulinum toxin is packaged in multiple portions, for example, 3-5 portions. In one embodiment, the botulinum toxin is botulinum toxin type A. In one embodiment, the kit of parts further comprises instructions for assessing or reviewing an assessment of a subject using a Masseter Muscle Prominence Scale (MMPS) prior to identifying the region of maximal bulge of the masseter muscle, instructions for identifying a region of maximal bulge of the masseter muscle, and/or instructions for repeating the identifying and the injecting on an opposing masseter muscle.

In another aspect, a kit of parts is provided that comprises: a) botulinum toxin in an amount of about 5-100 Units, and b) instructions for administration of botulinum toxin into a muscle for reducing prominence of the masseter muscle of a human, in which i) identifying a region of maximal bulge in the masseter muscle when the jaw is in a clenched position, and ii) when the jaw is in a relaxed position, administering to a plurality of sites in the region of the masseter muscle a dose of botulinum toxin that reduces prominence of the masseter muscle. In one embodiment, the amount of botulinum toxin is between about 10-50 Units or between 20-100 Units, preferably between about 12-48 Units or 24-96 Units, more preferably between about 24-36 Units or 48-72 Units. In a specific embodiment, the amount of botulinum toxin is 12 Units, 24 Units, 36 Units, 48 Units, 72 Units or 96 Units. In one embodiment, the botulinum toxin is botulinum toxin type A. In one embodiment, the botulinum toxin is packaged in multiple portions, preferably 2-5 portions, more preferably 3 portions.

In another aspect, a kit of parts is provided that comprises: a) botulinum toxin in an amount of about 5-100 Units, and b) instructions for administration of botulinum toxin into a muscle for temporarily reducing lower face convexity or width associated with masseter muscle prominence, in which i) identifying a line that extends from the lateral commissure of the mouth to the point where the ear lobe attaches to the face; ii) identifying a region of maximal bulge of the masseter muscle with the jaw in a clenched position; iii) determining a treatment area that includes the region of maximal bulge, is positioned at or below the line, is posterior to the risorius muscle and is anterior to the parotid gland; and iv) injecting botulinum toxin at a plurality of injection sites in the treatment area to administer a dose of botulinum toxin that reduces lower face convexity or width. In one embodiment, the amount of botulinum toxin is between about 10-50 Units or between 20-100 Units, preferably between about 12-48 Units or 24-96 Units, more preferably between about 24-36 Units or 48-72 Units. In a specific embodiment, the amount of botulinum toxin is 12 Units, 24 Units, 36 Units, 48 Units, 72 Units or 96 Units. In one embodiment, the botulinum toxin is packaged in multiple portions, preferably 2-5 portions, more preferably 3 portions. In one embodiment, the botulinum toxin is botulinum toxin type A. In one embodiment, the kit of parts further comprises instructions for assessing or reviewing an assessment of a subject using a Masseter Muscle Prominence Scale (MMPS), which assigns a rating to the subject of marked or very marked on the MMPS.

In another aspect, a kit of parts is provided that comprises: a) botulinum toxin in an amount of about 5-100 Units, and b) instructions for administration of botulinum toxin into a muscle for altering the contour of a lower face of a human, in which i) identifying a line that extends from the lateral commissure of the mouth to the point where the ear lobe attaches to the face; ii) identifying a region of maximal bulge of the masseter muscle with the jaw in a clenched position; iii) determining a treatment area that includes the region of maximal bulge, is positioned at or below the line, is posterior to the risorius muscle and is anterior to the parotid gland; and iv) injecting botulinum toxin at a plurality of injection sites in the treatment area to administer a dose of botulinum toxin that reduces lower face convexity or width. In one embodiment, the amount of botulinum toxin is between about 10-50 Units or between 20-100 Units, preferably between about 12-48 Units or 24-96 Units, more preferably between about 24-36 Units or 48-72 Units. In a specific embodiment, the amount of botulinum toxin is 12 Units, 24 Units, 36 Units, 48 Units, 72 Units or 96 Units. In one embodiment, the botulinum toxin is packaged in multiple portions, preferably 2-5 portions, more preferably 3 portions. In one embodiment, the botulinum toxin is botulinum toxin type A. In another aspect, a kit of parts is provided. The kit of parts comprises: a) botulinum toxin in an amount of about 5-100 Units, and b) instructions for administration of botulinum toxin into a muscle for sculpting or reshaping a lower face of a human in the masseter muscle region. In one embodiment, the amount of botulinum toxin is between about 10-50 Units or between 20-100 Units, preferably between about 12-48 Units or 24-96 Units, more preferably between about 24-36 Units or 48-72 Units. In a specific embodiment, the amount of botulinum toxin is 12 Units, 24 Units, 36 Units, 48 Units, 72 Units or 96 Units. In one embodiment, the botulinum toxin is packaged in multiple portions, preferably 2-5 portions, more preferably 3 portions. In one embodiment, the botulinum toxin is botulinum toxin type A.

### EXAMPLES

The following non-limiting examples provide those of ordinary skill in the art with specific preferred methods to treat conditions within the scope of embodiments of the present invention and are not intended to limit the scope of the invention.

### Materials and Methods:

Lower facial volume was quantified using computer tomography. A measurement selection area created by the perimeter (C-F-D-E) formed using anatomical landmarks: lateral canthus (A), alar recess (B), earlobe attachment point (C), prejowl sulcus (D), and mandible point (E). A single interpolated landmark (F) was also used at the intersection point of surface lines between points (A)-(D) and (B)-(C). The difference in lower facial volume between two 3D surface models of the same subject at different time points, for example baseline (pre-treatment) and post-treatment, was used to determine the change in facial volume. The lower facial volume is the summed volumes for both the left side and the right side of the face, which were compared between each paired baseline/post-treatment time point for the study. Lower facial volume was performed using the VECTRA M3 3D Stereophotogrammetry system digital photography system (Canfield Scientific, Inc., Fairfield, NJ, USA). The changes in lower facial volume quantified by VECTRA was also confirmed by CT evaluation.

### EXAMPLE 1

### TREATMENT OF MMH

A double-blind, placebo controlled, randomized study was performed to evaluate the efficacy and safety of a range of doses of a botulinum toxin Type A for treatment of subjects with masseter muscle hypertrophy (MMH). Potential subjects for treatment were assessed using the Masseter Muscle Prominence Scale (MMPS) and digital photography to determine a lower facial volume. Subjects rated as Grade 4 "marked" or Grade 5 "very marked" MMH were enrolled in the study. 187 subjects were enrolled and were randomized into 4 treatment cohorts for treatment with botulinum toxin Type A (BOTOX^{®}) according to the doses in Table 1-1.

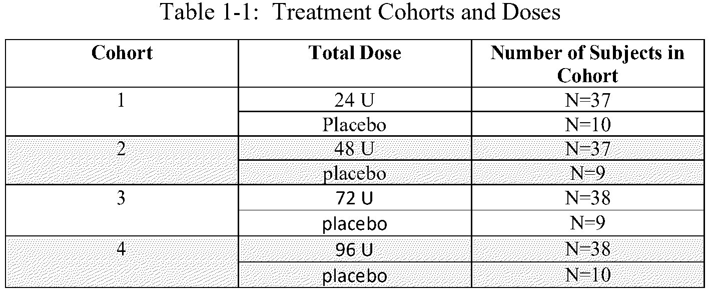

The indicated total dose was administered bilaterally and intramuscularly to each masseter muscle of each subject in each cohort. Three injections were made into each masseter muscle, for a total of six injections for bilateral treatment. For example, each subject in cohort 1 was treated with total dose of 24 Units, the total dose divided equally for bilateral treatment to administer 12 Units per masseter muscle. The 12 Unit dose per masseter muscle was divided equally into 3 injection sites per masseter muscle. Each subject in cohort 4 was treated with total dose of 96 Units, the total dose divided equally for bilateral treatment with 48 Units per masseter muscle. The 48 Unit dose per masseter muscle was divided equally into 3 injection sites per masseter muscle. The 3 injection sites in each masseter muscle were within a treatment area at or below a line that extended from the lateral commissure of the mouth to the point where the ear lobe attaches to the face, that included the region of maximal bulge of the masseter muscle, and posterior to the risorius muscle and anterior to the parotid gland.

Each subject was evaluated monthly after treatment for a one year period. Of the 187 subjects enrolled, 167 completed the 1 year study. Of the 20 patients that did not complete the study, the majority (13/20) discontinued for personal reasons. Only one discontinuation was due to an adverse event by a subject in the placebo group. At each monthly visit lower facial volume (cm³) was calculated using a three-dimensional imaging system (VECTRA M3, Canfield Scientific, Inc.). Results are shown in FIGS. 2-5. Briefly, lower facial volume was significantly reduced for all dose groups versus placebo (p<.001) at Day 90 with significant volume reduction (p ≤.05) maintained for at least 6 months after each of two treatments with 48U, 72U or 96U. The changes in lower facial volume quantified by VECTRA was confirmed by CT evaluations. The proportion or responders achieving MMPS Grade 3 and ≥2-grade change at Day 90 was significant for all dose groups versus placebo (p<.001). Significant difference (p ≤.05) was maintained at all time points through Day 180 with 48U, 72U, or 96U doses, compared with placebo. Reduction of MMH severity was sustained for over 9 months after a single dose of 72 U or 96U. The most frequent treatment related adverse effects was mastication disorder (BOTOX 5.3%, placebo 2.7%), mainly reported as mild chewing weakness. Treatments were well tolerated with no clinically-relevant correlations between adverse effect incidence and dose. No abnormally clinically-relevant changes were observed by CT scan or dental exams.

### EXAMPLE 2

### TREATMENT OF MMH

The subjects enrolled in the study of Example 1 were evaluated at day 180 of the study and eligible for a second treatment (retreatment) if, based on a clinician's assessment using the MMPS the subject had marked (Grade 4) or very marked (Grade 5) bilateral masseter muscle hypertrophy. For the subjects eligible for retreatment, the second treatment was given at the day 180 visit at the same dose level given at the beginning (day 1) of the study. The subjects that were retreated were evaluated at monthly intervals for 180 days to determine lower facial volume (cm³) using a three-dimensional imaging system (VECTRA M3, Canfield Scientific, Inc.). The changes in lower facial volume quantified by VECTRA was confirmed by CT evaluation. Results are shown in FIG. 6.

Many alterations and modifications may be made by those having ordinary skill in the art, without departing from the spirit and scope of the disclosure. Therefore, it must be understood that the described embodiments have been set forth only for the purposes of examples, and that the embodiments should not be taken as limiting the scope of the following claims. The following claims are, therefore, to be read to include not only the combination of elements which are literally set forth, but all equivalent elements for performing substantially the same function in substantially the same way to obtain substantially the same result. The claims are thus to be understood to include those that have been described above, those that are conceptually equivalent, and those that incorporate the ideas of the disclosure.

### EMBODIMENTS

1. A method to reduce lower face width in a masseter muscle region, comprising:
   identifying a region of maximal bulge of the masseter muscle; and
   injecting botulinum toxin at a plurality of injection sites in the region to administer a dose of botulinum toxin to reduce lower face width in the masseter muscle region.
2. The method of embodiment 1, wherein said identifying and said injecting is performed bilaterally.
3. The method of embodiment 1, further comprising repeating said identifying and said injecting on an opposing masseter muscle.
4. The method of any one of embodiments 1-3, wherein injecting comprises injecting a dose of botulinum toxin in a range selected from the group of ranges of between about 5-100 Units, between about 15-60 Units, between about 25-47 Units, and between about 30-40 Units to each of the muscles treated, or wherein the dose is about 36 Units, to each of the muscles treated.
5. The method of any one of embodiments 1-3, wherein injecting comprises injecting a dose of botulinum toxin that is between about 24-72 Units.
6. The method of any one of embodiments 1-5, wherein the plurality of injection sites is between 3-5 injection sites.
7. The method of any one of embodiments 1-6, wherein a portion of the dose is administered at each injection site in the plurality of injection sites, where the portion is equal to the dose divided by the number of sites in the plurality of injection sites.
8. The method of any one of embodiments 1-7, further comprising, prior to said identifying, assessing or reviewing an assessment of a subject using a Masseter Muscle Prominence Scale (MMPS).
9. The method of embodiment 8, wherein said assessing or reviewing assigns a rating to the subject of marked or very marked on the MMPS.
10. The method of embodiment 8, wherein the assessing or reviewing is carried out to evaluate the efficacy of a previous treatment.
11. The method of any one of embodiments 1 -9, wherein the method is a cosmetic method to reduce lower face width in a masseter muscle region.
12. A method to reduce prominence of the masseter muscle of a human, comprising:
   administering to a region of the masseter muscle a dose of botulinum toxin that reduces prominence of the masseter muscle, wherein the dose is administered to a plurality of sites in the region of the masseter muscle; wherein the region is identified as a region of maximal bulge in the masseter muscle when the jaw is in a clenched position; and
   wherein the dose is administered with the jaw in a relaxed position.
13. The method of embodiment 12, wherein said administering is performed bilaterally.
14. The method of embodiment 12, wherein said administering comprises administering a dose of botulinum toxin of between about 5-100 Units, about 15-60 Units, about 25-47 Units, about 30-40 Units, or about 36 Units to each of the muscles treated.
15. The method of embodiment 12, further comprising repeating said administering on an opposing masseter muscle of the human.
16. The method of any one of embodiments 12-15, wherein administering is by injection with a needle positioned during injecting to be perpendicular to the masseter muscle.
17. The method of any one of embodiments 12-16, wherein the dose is administered in a manner for distribution to deep muscle and to superficial muscle.
18. The method of any one of embodiments 12-17, wherein the dose is administered in a volume of between about 0.6-2.4 mL.
19. The method of any one of embodiments 12-17, wherein a portion of the dose is administered at each injection site in the plurality of injection sites, where the portion administered at each injection site is unequal.
20. The method of embodiment 19, wherein the portion is administered in a volume of between about 0.1-0.4 mL.
21. The method of any one of embodiments 12-20, wherein the plurality of injection sites is 3 injection sites.
22. The method of any one of embodiments 12-21, further comprising, prior to said administering, selecting a human subject using an MMPS.
23. The method of any one of embodiments 12-22, wherein the method is a cosmetic method to reduce prominence of the masseter muscle of a human.
24. A method to temporarily reduce lower face convexity or width associated with masseter muscle prominence, comprising:
   (i) identifying a line that extends from the lateral commissure of the mouth to the point where the ear lobe attaches to the face;
   (ii) identifying a region of maximal bulge of the masseter muscle with the jaw in a clenched position;
   (iii) determining a treatment area that includes the region of maximal bulge, is positioned at or below the line, is posterior to the risorius muscle and is anterior to the parotid gland; and
   (iv) injecting botulinum toxin at a plurality of injection sites in the treatment area to administer a dose of botulinum toxin that reduces lower face convexity or width.
25. The method of embodiment 24, wherein steps (i)-(iv) are performed bilaterally.
26. The method of embodiment 24 or embodiment 25, wherein injecting comprises injecting a dose of botulinum toxin of between about 5-100 Units, about 15-60 Units, about 25-47 Units, about 30-40 Units, or about 36 Units, to each of the muscles treated.
27. The method of embodiment 24, further comprising (v) repeating steps (i)-(iv) on an opposing masseter muscle of the face.
28. The method of any one of embodiments 24-27, wherein step (i) comprises visually identifying an imaginary line.
29. The method of any one of embodiments 24-27, wherein step (i) comprises marking the line on skin of the face.
30. The method of any one of embodiments 24-29, wherein step (iii) comprises visually determining the treatment area without marking skin.
31. The method of any one of embodiments 24-29, wherein step (iii) comprises determining the treatment area and marking on the skin the treatment area.
32. The method of any one of embodiments 24-31, further comprising, prior to step (i), assessing or reviewing an assessment of a subject using a Masseter Muscle Prominence Scale (MMPS).
33. The method of embodiment 32, wherein said assessing or reviewing assigns a rating to the subject of marked or very marked on the MMPS.
34. The method of embodiment 32 or embodiment 33, wherein the assessing or reviewing an assessment of a subject using the MMPS is carried out to evaluate the efficacy of a previous treatment.
35. The method of any one of embodiments 24-34, wherein the method is a cosmetic method to temporarily reduce lower face convexity or width associated with masseter muscle prominence.
36. The method of any preceding embodiment, wherein the botulinum toxin is botulinum toxin type A.
37. The method of any preceding embodiment, wherein the botulinum toxin is animal protein free.
38. The method of embodiment 37, wherein the lower face width in a masseter muscle region, the prominence of the masseter muscle of a human, or the lower face convexity or width associated with masseter muscle prominence is reduced for a period of time longer than obtained with an animal-protein containing composition.
39. A cosmetic method to reduce lower face width in a masseter muscle region, comprising:
   identifying a region of maximal bulge of the masseter muscle; and injecting botulinum toxin at a plurality of injection sites in the region to administer a dose of botulinum toxin to reduce lower face width in the masseter muscle region,
   wherein injecting comprises injecting a dose of botulinum toxin of between about 15-60 Units, about 25-47 Units, about 30-40 Units, or about 36 Units to each of the muscles treated.
40. A cosmetic method to reduce prominence of the masseter muscle of a human, comprising:
   administering to a region of the masseter muscle a dose of botulinum toxin that reduces prominence of the masseter muscle, wherein the dose is administered to a plurality of sites in the region of the masseter muscle;
   wherein the region is identified as a region of maximal bulge in the masseter muscle when the jaw is in a clenched position;
   wherein the dose is administered with the jaw in a relaxed position, and
   wherein administering comprises administering a dose of botulinum toxin of between about 15-60 Units, about 25-47 Units, about 30-40 Units, or about 36 Units to each of the muscles treated.
41. A cosmetic method to temporarily reduce lower face convexity or width associated with masseter muscle prominence, comprising:
   identifying a line that extends from the lateral commissure of the mouth to the point where the ear lobe attaches to the face;
   identifying a region of maximal bulge of the masseter muscle with the jaw in a clenched position;
   determining a treatment area that includes the region of maximal bulge, is positioned at or below the line, is posterior to the risorius muscle and is anterior to the parotid gland; and
   injecting botulinum toxin at a plurality of injection sites in the treatment area to administer a dose of botulinum toxin that reduces lower face convexity or width, wherein injecting comprises injecting a dose of botulinum toxin of about 15-60 Units, about 25-47 Units, about 30-40 Units, or about 36 Units to each of the muscles treated.
42. The method of any one of embodiments 39-41, wherein the lower face width in a masseter
   muscle region, the prominence of the masseter muscle of a human, or the lower face convexity or width associated with masseter muscle prominence is reduced for a period of time longer than with a dosage of less than or more than that specified.
43. A kit of parts, comprising:
   a) botulinum toxin in an amount of about 5-100 Units, and
   b) instructions for administration of botulinum toxin into a muscle for the treatment of masseter muscle hypertrophy, in which administering botulinum toxin into the masseter muscle of a subject having masseter muscle hypertrophy is useful for treatment.
44. The kit of parts according to embodiment 43, wherein the instructions include instructions to
   administer bilaterally.
45. The kit of parts according to embodiment 43 or embodiment 44, wherein the instructions include instructions to administer a dose of botulinum toxin in a range selected from the group of ranges of between about 15-60 Units, between about 24-72 Units, between about 25-47 Units, between about 30-40 Units, or about 36 Units, to each of the muscles treated.
46. The kit of parts according to any one of embodiments 43-45, wherein the instructions include instructions to administer a dose of botulinum toxin at a plurality of injection sites between 3-5 injection sites.
47. The kit of parts according to embodiment 46, wherein a portion of the dose is administered at each injection site in the plurality of injection sites, where the portion is equal to the dose divided by the number of sites in the plurality of injection sites.
48. The kit of parts according to any one of embodiments 43-47, wherein the instructions include instructions to provide and/or review an assessment of a subject using a Masseter Muscle Prominence Scale (MMPS).
49. The kit of parts according to embodiment 48, wherein the MMPS assigns a rating to the subject of marked or very marked to the subject.
50. A kit of parts, comprising:
   a) botulinum toxin in an amount of about 5-100 Units, and
   b) instructions for administration of botulinum toxin into a muscle for reducing lower face width in a masseter muscle region, in which
      i) identifying a region of maximal bulge of the masseter muscle, and
      ii) injecting botulinum toxin at a plurality of injection sites in the region to administer a dose of botulinum toxin to reduce lower face width in the masseter muscle region.
51. The kit of parts according to embodiment 44, wherein said identifying and said injecting is performed bilaterally.
52. The kit of parts according to embodiment 44 or embodiment 51, further comprising repeating said identifying and said injecting on an opposing masseter muscle.
53. The kit of parts according to any one of embodiments 44-52, wherein injecting comprises injecting a dose of botulinum toxin in a range selected from the group of ranges of between about 15-60 Units, between about 25-47 Units, between about 30-40 Units, or about 36 Units, to each of the muscles treated.
54. The kit of parts according to any one of embodiments 44-52, wherein injecting comprises injecting a dose of botulinum toxin that is between about 24-72 Units.
55. The kit of parts according to any one of embodiments 44-54, wherein the plurality of injection sites is between 3-5 injection sites.
56. The kit of parts according to any one of embodiments 44-55, wherein a portion of the dose is administered at each injection site in the plurality of injection sites, where the portion is equal to the dose divided by the number of sites in the plurality of injection sites.
57. The kit of parts according to any one of embodiments 44-56, further comprising, prior to said identifying, assessing or reviewing an assessment of a subject using a Masseter Muscle Prominence Scale (MMPS).
58. The kit of parts according to embodiment 57, wherein said assessing or reviewing assigns a rating to the subject of marked or very marked on the MMPS.
59. A kit of parts, comprising:
   a) botulinum toxin in an amount of about 5-100 Units, and
   b) instructions for administration of botulinum toxin into a muscle for reducing prominence of the masseter muscle of a human, in which
      i) identifying a region of maximal bulge in the masseter muscle when the jaw is in a clenched position, and
      ii) when the jaw is in a relaxed position, administering to a plurality of sites in the region of the masseter muscle a dose of botulinum toxin that reduces prominence of the masseter muscle.
60. The kit of parts according to embodiment 59, wherein said administering is performed
   bilaterally.
61. The kit of parts according to any one of embodiments 59-60, wherein said administering comprises administering a dose of botulinum toxin of between about 5-100 Units, about 15-60 Units, about 25-47 Units, about 30-40 Units, or about 36 Units to each of the muscles treated.
62. The kit of parts according to any one of embodiments 59-61, wherein the instructions further provide instructions to repeat said administering on an opposing masseter muscle of the human.
63. The kit of parts according to any one of embodiments 59-62, wherein the administering is by injection with a needle positioned during injecting to be perpendicular to the masseter muscle.
64. The kit of parts according to any one of embodiments 59-63, wherein the botulinum toxin is administered in a manner for distribution to deep muscle and to superficial muscle.
65. The kit of parts according to any one of embodiments 59-64, wherein the botulinum toxin is administered in a volume of between about 0.6-2.4 mL.
66. The kit of parts according to any one of embodiments 59-65, wherein a portion of a dose of the botulinum toxin is administered at each injection site in the plurality of injection sites, where theportion administered at each injection site is unequal.
67. The kit of parts according to any one of embodiments 59-66, wherein the portion is administered in a volume of between about 0.1-0.4 mL.
68. The kit of parts according to any one of embodiments 59-67, wherein the plurality of injection sites is 3 injection sites.
69. The kit of parts according to any one of embodiments 59-68, further comprising, prior to said administering, selecting a human subject using an MMPS.
70. The kit of parts according to any one of embodiments 59-69, wherein the method is a cosmetic method to reduce prominence of the masseter muscle of a human.
71. A kit of parts, comprising:
   a) botulinum toxin in an amount of about 5-100 Units, and
   b) instructions for administration of botulinum toxin into a muscle for temporarily reducing lower face convexity or width associated with masseter muscle prominence, in which
      i) identifying a line that extends from the lateral commissure of the mouth to the point where the ear lobe attaches to the face;
      ii) identifying a region of maximal bulge of the masseter muscle with the jaw in a clenched position;
      iii) determining a treatment area that includes the region of maximal bulge, is positioned at or below the line, is posterior to the risorius muscle and is anterior to the parotid gland; and
      iv) injecting botulinum toxin at a plurality of injection sites in the treatment area to administer a dose of botulinum toxin that reduces lower face convexity or width.
72. The kit of parts according to embodiment 71, wherein steps (i)-(iv) are performed bilaterally.
73. The kit of parts according to embodiment 71 or embodiment 72, wherein injecting comprises injecting a dose of botulinum toxin of between about 5-100 Units, about 15-60 Units, about 25-47 Units, about 30-40 Units, or about 36 Units to each of the muscles treated.
74. The kit of parts according to any one of embodiments 71-73, further comprising (v) repeating steps (i)-(iv) on an opposing masseter muscle of the face.
75. The kit of parts according to any one of embodiments 71-74, wherein step (i) comprises visually identifying an imaginary line.
76. The kit of parts according to any one of embodiments 71-74, wherein step (i) comprises marking the line on skin of the face.
77. The kit of parts according to any one of embodiments 71-76, wherein step (iii) comprises visually determining the treatment area without marking skin.
78. The kit of parts according to any one of embodiments 71-76, wherein step (iii) comprises
   determining the treatment area and marking on the skin the treatment area.
79. The kit of parts according to any one of embodiments 71-78, further comprising, prior to step
   (i), assessing or reviewing an assessment of a subject using a Masseter Muscle Prominence Scale (MMPS).
80. The kit of parts according to embodiment 79, wherein said assessing or reviewing assigns a rating to the subject of marked or very marked on the MMPS.
81. The kit of parts according to embodiment 79, wherein the assessing or reviewing an assessment of a subject using the MMPS is carried out to evaluate the efficacy of a previous treatment.
82. The kit of parts according to any one of embodiments 43-81, wherein the kit is for a cosmetic treatment.
83. The kit of parts according to any one of embodiments 43-82, wherein the botulinum toxin is botulinum toxin type A.
84. The kit of parts according to any one of embodiments 43-83, wherein the botulinum toxin is animal protein free.
85. The kit of parts according to embodiment 84, wherein the lower face width in a masseter muscle region, the prominence of the masseter muscle of a human, or the lower face convexity or width associated with masseter muscle prominence is reduced for a period of time longer than obtained with an animal-protein containing composition.
86. A composition comprising botulinum toxin and a pharmaceutically acceptable carrier for injection into a region of maximal bulge of the masseter muscle to reduce lower face width in the masseter muscle region of a subject.
87. The composition according to embodiment 86, wherein the subject has marked or very marked masseter muscle prominence.
88. The composition according to embodiment 87, wherein the masseter muscle prominence is determined using the MMPS.
89. The composition according to any one of embodiments 86-88, wherein the composition provides a reduction in lower face width in the masseter muscle region for 90 days after injection.
90. The composition according to any one of embodiments 86-89, wherein the composition is injected bilaterally to the masseter muscles at a plurality of injection sites at each masseter muscle.
91. The composition according to embodiment 90, wherein the plurality of injection sites is between 3-5 per masseter muscle.
92. The composition according to embodiment 90 or embodiment 91, wherein a dose of the composition for injection is between about 5-100 Units, about 15-60 Units, about 25-47 Units, about 30-40 Units, or about 36 Units to each of the muscles treated.
93. The composition according to any one of embodiments 86-92, wherein the composition is injected with a needle positioned during injecting to be perpendicular to the masseter muscle.
94. The composition according to any one of embodiments 86-93, wherein the composition is injected in a manner for distribution to deep muscle and to superficial muscle.
95. The composition according to any one of embodiments 86-94, wherein the composition is for a cosmetic treatment.
96. The composition according to any one of embodiments 86-95, wherein the botulinum toxin is botulinum toxin type A.
97. The composition according to any one of embodiments 86-96, wherein the botulinum toxin is animal protein free.
98. The composition according to embodiment 97, wherein the lower face width in a masseter muscle region, the prominence of the masseter muscle of a human, or the lower face convexity or width associated with masseter muscle prominence is reduced for a period of time longer than obtained with an animal-protein containing composition.
99. Use of botulinum toxin for injection into a region of maximal bulge of the masseter muscle to reduce lower face width in the masseter muscle region of a subject, to reduce prominence of the masseter muscle of a human, and/or to temporarily reduce lower face convexity or width associated with the masseter muscle prominence a subject.

## Claims

1. A cosmetic method to reduce prominence of the masseter muscle of a human, comprising:
(i) administering to a region of the masseter muscle a dose of botulinum toxin that reduces prominence of the masseter muscle, wherein the dose is administered to a plurality of sites in the region of the masseter muscle;
wherein the region is identified as a region of maximal bulge in the masseter muscle when the jaw is in a clenched position; and wherein the dose is administered with the jaw in a relaxed position, and
(ii) repeating said administering on an opposing masseter muscle of the human, wherein said administering comprises administering a dose of botulinum toxin of 24 Units to each of the muscles treated,
wherein the plurality of injection sites is 3 injection sites, and
wherein the botulinum toxin is onabotulinum toxin type A.

2. The cosmetic method of claim 1, wherein administering is by injection with a needle positioned during injecting to be perpendicular to the masseter muscle.

3. The cosmetic method of any one of claims 1-2, wherein the dose is administered in a manner for distribution to deep muscle and to superficial muscle.

4. The cosmetic method of any one of claims 1-3, wherein the dose is administered in a volume of between about 0.6-2.4 mL.

5. The cosmetic method of any one of claims 1-4, wherein a portion of the dose is administered at each injection site in the plurality of injection sites, where the portion is equal to the dose divided by the number of sites in the plurality of injection sites.

6. The cosmetic method of claim 5, wherein the portion is administered in a volume of between about 0.1 - 0.4 mL.
